(19) Europäisches Patentamt
European Patent Office
Office européen des brevets

(11) **EP 2 122 364 B1**

(12) **EUROPÄISCHE PATENTSCHRIFT**

(45) Veröffentlichungstag und Bekanntmachung des Hinweises auf die Patenterteilung:
**21.11.2012 Patentblatt 2012/47**

(21) Anmeldenummer: **08707734.3**

(22) Anmeldetag: **15.02.2008**

(51) Int Cl.:
*G01N 33/68* (2006.01)    *G01N 33/58* (2006.01)

(86) Internationale Anmeldenummer:
**PCT/EP2008/001161**

(87) Internationale Veröffentlichungsnummer:
**WO 2008/110247 (18.09.2008 Gazette 2008/38)**

(54) **VERFAHREN ZUR IDENTIFIZIERUNG DER AGONISTISCHEN AKTIVITÄT EINER ZIELVERBINDUNG AUF EINEN KALIUMKANAL**

METHOD FOR IDENTIFYING THE AGONISTIC ACTIVITY OF A TARGET COMPOUND ON A POTASSIUM CHANNEL

PROCÉDÉ D'IDENTIFICATION DE L'ACTIVITÉ AGONISTE D'UN COMPOSÉ CIBLE SUR UN CANAL POTASSIQUE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **13.03.2007 DE 102007012029**

(43) Veröffentlichungstag der Anmeldung:
**25.11.2009 Patentblatt 2009/48**

(73) Patentinhaber: **Grünenthal GmbH**
**52078 Aachen (DE)**

(72) Erfinder:
• **STRÜNKER, Timo**
**50937 Köln (DE)**
• **CAVALAR, Markus**
**23923 Herrnburg (DE)**
• **BAHRENBERG, Gregor**
**52078 Aachen (DE)**

(74) Vertreter: **Hiebl, Inge Elisabeth**
**Kraus & Weisert**
**Patent- und Rechtsanwälte**
**Thomas-Wimmer-Ring 15**
**80539 München (DE)**

(56) Entgegenhaltungen:
**EP-A- 1 760 158       WO-A-02/32419**
**US-A1- 2004 175 691      US-B1- 6 329 367**

• **WOLFF C ET AL: "COMPARATIVE STUDY OF MEMBRANE POTENTIAL-SENSITIVE FLUORESCENT PROBES AND THEIR USE IN ION CHANNEL SCREENING ASSAYS" JOURNAL OF BIOMOLECULAR SCREENING, LARCHMONT, NY, US, Bd. 8, Nr. 5, 2003, Seiten 533-543, XP008055803 ISSN: 1087-0571**

**Beschreibung**

Die vorliegende Erfindung betrifft ein Verfahren bzw. einen Assay zur Identifizierung der agonistischen Aktivität einer Zielverbindung auf einen spannungsabhängigen Kaliumionenkanal.

Ionenkanäle sind für die Funktion von Nerven- und Muskelzellen sowie der meisten anderen somatischen Zellen essentiell. Ionenkanäle sind an nahezu allen physiologischen Vorgängen beteiligt, zum Beispiel an den elektrischen Impulsen, die den sensorischen und motorischen Funktionen im Gehirn zugrunde liegen, an der Kontrolle der kontraktilen Aktivität des Herzens, der glatten Muskulatur in Skelettgefäßen und Eingeweiden sowie bei der Nährstoffaufnahme, Hormonsekretion, Zellsekretion und Zellentwicklung.

Ionenkanäle ermöglichen Ionen, die hydrophobe Lipiddoppelschicht der Zellmembran zu passieren, was entscheidend für die Entstehung und Weiterleitung elektrischer Signale in lebenden Zellen ist. Ionenkanäle können anhand ihrer Ionenselektivität klassifiziert werden. Einige Ionenkanäle sind nur für eine ganz bestimmte Ionensorte durchlässig, zum Beispiel für Natrium- ($Na^+$), Kalium- ($K^+$) oder für Kalziumionen ($Ca^{2+}$). Andere Kanäle wiederum können nicht oder nur schlecht zwischen verschiedenen Ionensorten unterscheiden. Sie werden als nichtselektive Ionenkanäle bezeichnet. Das Öffnen oder Schließen von Ionenkanälen kann durch unterschiedliche Mechanismen gesteuert werden. Ligandenabhängige Ionenkanäle werden zum Beispiel durch die direkte Bindung intra- oder extrazellulärer Liganden kontrolliert. Spannungsabhängige Ionenkanäle reagieren dagegen auf Änderungen des Membranpotenzials.

Das Membranpotenzial einer Zelle wird aus dem elektrischen Gradienten und dem Konzentrationsgradienten verschiedener Ionen und geladener Teilchen beiderseits der Zellmembran hervorgerufen. Von besonderer Bedeutung ist dabei die Verteilung von Natrium- und Kaliumionen im intra- und extrazellulären Medium der Zelle. Die Ionenverteilung beruht auf der selektiven Permeabilität der Membran für die verschiedenen Ionen und dem aktiven Transport von Ionen mit Hilfe von Ionenpumpen.

Die Aktivität dieser ATP-getriebenen "Pumpen" in der Zelle führt zu einer Ungleichverteilung verschiedener Ionen - vor allem $K^+$, $Na^+$, $Ca^{2+}$, $Cl^-$ und organischer Anionen - beiderseits der Zellmembran und so zu Ionengradienten über die Membran. Im Ruhezustand befindet sich dadurch ein Überschuss an negativen Ladungen auf der Innenseite der Membran im Vergleich zur Außenseite der Membran. Diese Ladungstrennung führt zu einer Differenz im elektrischen Potenzial über der Zellmembran, das als Membranpotenzial ($V_m$) bezeichnet wird.

Im Ruhezustand liegt das Membranpotenzial bei Neuronen und Muskelzellen im Bereich von -60 mV bis -80 mV (sog. Ruhemembranpotenzial). Elektrische Signale in Neuronen und Muskelzellen beruhen auf kurzfristigen Änderungen des Membranpotenzials. Diese Änderungen werden hervorgerufen durch transientes Öffnen und Schließen von Ionenkanälen, was zu dem Fluss von elektrischem Strom über die Membran führt. Wird dabei die Ladungstrennung an der Membran verringert, spricht man von einer Depolarisation der Membran - das Membranpotenzial wird weniger negativ. Eine Erhöhung der Ladungstrennung wird als Hyperpolarisation bezeichnet, da sich ein Membranpotenzial negativer als das Ruhemembranpotenzial einstellt.

Das Ruhemembranpotenzial ist ein sog. Diffusionspotential und wird durch verschiedene Faktoren bestimmt: Ungeladene Moleküle wie z.B. Sauerstoff, Kohlendioxid, Harnstoff etc. können die hydrophobe Lipiddoppelschicht der Zellmembran ungehindert passieren. Ionen, geladene Teilchen, können die Zellmembran dagegen nur mit Hilfe von selektiven, proteinbasierten Poren (Ionenkanäle) überqueren. Aufgrund ihrer selektiven Permeabilität für bestimmte Ionenspezies entspricht eine Zellmembran einer semipermeablen Membran. Die Aktivität von ATP-getriebenen "Pumpen" in der Membran einer Zelle führt zu einer Ungleichverteilung verschiedener Ionen - vor allem $K^+$, $Na^+$, $Ca^{2+}$, $Cl^-$ und organischer Anionen - beiderseits der Zellmembran und so zu Ionengradienten über die Membran. In Tabelle 1 ist die intrazelluläre und die extrazelluläre Konzentration der wichtigen Ionensorten dargestellt. Die Semipermabilität der Zellmembran und die Ungleichverteilung von Ionen beiderseits der Zellmembran, führen zur Entstehung des Ruhemembranpotenzials.

**Tabelle 1: Konzentration der wichtigsten Ionensorten innerhalb und außerhalb einer Zelle**

| Ion | extrazelluläre Konzentration (mM) | intrazelluläre Konzentration (mM) |
|---|---|---|
| $Na^+$ | 145 | 12 |
| $K^+$ | 4 | 155 |
| $Ca^{2+}$ | 1,5 | 0,0001 |
| $Cl^-$ | 123 | 4,2 |

Um diesen Zusammenhang zu verdeutlichen, wird vereinfacht zunächst eine Zelle betrachtet, deren Membran nur für eine Ionensorte permeabel ist, z.B. für Kaliumionen ($K^+$). $K^+$-Ionen liegen in viel größerer Konzentration innerhalb einer Zelle vor, verglichen mit ihrer Umgebung. Deshalb diffundieren $K^+$-Ionen entlang ihres chemischen Gradienten vom Inneren der Zelle nach außen. Die Diffusion von K$^+$ aus der Zelle heraus ist allerdings selbstlimitierend. Der Ausstrom der positiv geladen K$^+$-Ionen und der daraus resultierende Überschuss an impermeablen, negativ geladenen Anionen in der Zelle führen dazu, dass das Innere der Zelle negativ wird. Aufgrund dieser Ladungstrennung entwickelt sich eine elektrische Potenzialdifferenz über der Zellmembran. Je mehr K$^+$-Ionen nun die Zelle verlassen, desto größer wird die Ladungstrennung und desto größer wird die Potenzialdifferenz, das Innere der Zelle wird immer negativer. Diese elektrische Potenzialdifferenz wirkt schließlich dem weiteren Ausstrom der positiv geladenen K-Ionen entgegen.

Das bedeutet, dass die Diffusion von Kalium über die Zellmembran von zwei entgegengesetzten Kräften beeinflusst wird: a) der chemischen Triebkraft, die von der Konzentrationsdifferenz für Kalium abhängt und b) der elektrischen Triebkraft, die durch die elektrische Potenzialdifferenz über der Membran bestimmt wird.

Die Diffusion von Kalium führt somit nach kurzer Zeit zu einem Membranpotenzial, an dem die chemische Triebkraft, die K$^+$-Ionen aus der Zelle hinaus befördert, exakt gleich groß der elektrischen Triebkraft des Membranpotenzials ist, die dem Ausstrom von K$^+$-Ionen entgegenwirkt. Genau dann, wenn dieses Membranpotenzial erreicht ist, findet kein Netto-Strom mehr von K$^+$-Ionen über die Membran statt. Dieses Potenzial wird als sog. "Kalium-Gleichgewichtspotenzial" ($E_K$) bezeichnet.

$$E_K = 61{,}4 \text{ mV} \times \log \left[ \frac{[K^+]_a}{[K^+]_i} \right]$$

**Gleichung 1: Nernst-Gleichung für Kalium (bei 37°C)**
**[Ion] = Konzentration (a) extrazellulär (i) intrazellulär**

Mit Hilfe der sog. Nernst-Gleichung lässt sich das Gleichgewichtspotenzial für Kaliumionen berechnen.

Laut der Nernst-Gleichung (Gleichung 1) wird das Kalium-Gleichgewichtspotenzial von dem Quotienten der Kaliumkonzentrationen beiderseits der Zellmembran bestimmt. Verwendet man die Kaliumkonzentrationen, die in Tabelle 1 angegeben sind, so ergibt sich ein Kalium-Gleichgewichtspotenzial von -98 mV. Das Membranpotenzial einer Zelle, deren Membran selektiv permeabel für Kalium ist, würde somit genau $E_K$ entsprechen und bei -98 mV liegen.

**Tabelle 2: Extrazelluläre und intrazelluläre Konzentration der wichtigsten Ionensorten (vgl. Tabelle 1) und daraus resultierendes Gleichgewichtspotenzial**

| Ion | extrazelluläre Konzentration (mM) | intrazelluläre Konzentration (mM) | Gleichgewichtspotenzial (mV) |
|---|---|---|---|
| Na$^+$ | 145 | 12 | +67 |
| K$^+$ | 4 | 155 | -98 |
| Ca$^{2+}$ | 1,5 | 0.0001 | +129 |
| Cl$^-$ | 123 | 4,2 | -90 |

In der Membran einer Zelle befinden sich in der Regel jedoch nicht nur Ionenkanäle, die Kalium leiten, sondern auch Kanäle für andere Ionenspezies. Wie in Tabelle 1 und Tabelle 2 dargestellt, gibt es über der Zellmembran auch einen Konzentrationsgradienten für diese Ionen. Folglich lässt sich auch für diese Ionen ein bestimmtes Gleichgewichtspotenzial ermitteln. In Tabelle 2 sind die Gleichgewichtspotenziale der wichtigsten Ionen dargestellt Der Gradient für Natriumionen liegt z.B. genau umgekehrt zu dem der Kaliumionen. Folglich liegt das Gleichgewichtspotenzial für Natrium mit +67mV weit im positiven Bereich.

Wie nun die verschiedenen Ionengradienten zum Ruhemembranpotenzial einer Zelle beitragen, die für Kalium-, Natrium- und Chloridionen permeabel ist, wird durch die sogenannte Goldman-Hodgkin-Katz Spannungs-Gleichung (Gleichung 2) beschrieben.

$$V_m = 61,4\,mV \, x \log \frac{P_K[K^+]_a + P_{Na}[Na^+]_a + P_{Cl}[Cl^-]_i}{P_K[K^+]_i + P_{Na}[Na^+]_i + P_{Cl}[Cl^-]_a}$$

**Gleichung 2: Goldman-Hodgkin-Katz Spannungsgleichung (37°C).**

[Ion] = Konzentration (a) extrazellulär (i) intrazellulär. $P_X$ = Permeabilität der Membran für Ion X.

$V_m$ = Membranpotenzial

Der Gleichung 2 lässt sich entnehmen, dass das Membranpotenzial einer Zelle nicht nur von den Quotienten der Ionenkonzentrationen beiderseits der Zellmembran bestimmt wird, sondern vor allem von der Permeabilität (P) der Membran für das jeweilige Ion. Je größer die Permeabilität (oft auch Leitfähigkeit genannt) der Membran für ein bestimmtes Ion ist, desto größer ist auch der Beitrag dieses Ions zum Membranpotenzial. Das Membranpotenzial stellt also gewissermaßen den gewichteten Mittelwert der Gleichgewichtspotenziale für die verschiedenen Ionen dar.

Die Permeabilität der Membran für die jeweilige Ionensorte wird allein durch die Anzahl und Aktivität der entsprechenden Ionenkanäle bestimmt, die dieses Ion leiten. Im Ruhezustand finden sich nur sehr wenig geöffnete Natrium-, Chlorid- oder Kalziumkanäle in der Zellmembran. Das Membranpotenzial der meisten Zellen wird in Ruhe hauptsächlich durch eine Kaliumleitfähigkeit der Membran bestimmt ($P_K$: $P_{Cl}$: $P_{Na}$ = 1:0,45:0,04). Deshalb haben Neuronen und Muskelzellen ein Ruhemembranpotenzial von -60 bis -80 mV (siehe oben), nahe dem Gleichgewichtspotenzial für Kalium.

Eine Änderung des Membranpotenzials einer Zelle erfolgt, wenn sich die Permeabilität der Membran für eine Ionensorte ändert. So verschiebt z.B. eine Aktivierung von Natriumkanälen das Membranpotenzial in Richtung des Gleichgewichtspotenzials für Natrium - das Membranpotenzial wird positiver.

Das Membranpotenzial kann auch einen neuen Wert annehmen, wenn sich die intrazelluläre oder extrazelluläre Konzentration eines Ions ändert (siehe Gleichung 2). Wie groß die Membranpotenzialänderung in diesem Fall ist, hängt davon ab, wie stark das Membranpotenzial insgesamt von diesem Ion bestimmt wird. Änderungen der extrazellulären Natrium-, Chlorid- oder Kalzium-Konzentration haben deshalb in der Regel nur einen geringen Einfluss auf das Membranpotenzial. Das Membranpotenzial wird dagegen sehr deutlich von der extrazellulären Kaliumkonzentration beeinflusst.

Das menschlichen Genom enthält etwa 50 verschiedene Kaliumkanal-Gene. Kaliumkanäle finden sich in nahezu jedem Zelltyp eines Organismus und stellen die größte und diverseste Ionenkanalfamilie dar. Die Aktivität wird je nach Kanaltyp durch unterschiedliche physiologische Stimuli moduliert, z.B. Membranpotenzialänderungen (spannungsabhängige Kanäle), G-Proteine, Kalziumionen, Nucleotide (ATP) etc.. In den meisten Zellen wird das Ruhemembranpotenzial durch Kaliumkanäle bestimmt (siehe oben). Kaliumkanäle beeinflussen in Neuronen und Muskelzellen entscheidend die Frequenz und den Zeitverlauf von Aktionspotenzialen, sowie deren Weiterleitung. Sie regulieren zudem die elektrische Erregbarkeit dieser Zellen und sie spielen eine entscheidende Rolle bei einigen sekretorischen Prozessen, wie z.B. der Insulinausschüttung.

Wirkstoffe können die Aktivität von Kalium-Kanälen auf unterschiedliche Art und Weise beeinflussen. Kanalblocker blockieren meist direkt die Kanalpore von der intrazellulären oder extrazellulären Seite der Membran.

Darüber hinaus kann z.B. aber auch die Wechselwirkung eines Kanalblockers mit einer akzessorischen Untereinheiten dazu führen, dass sich die Kanalpore schließt bzw. nicht durch physiologische Stimuli geöffnet werden kann (z.B. $K_{ATP}$-Kanal Blocker).

Kanalöffner können ebenfalls über eine Wechselwirkung mit akzessorischen Untereinheiten zu einem Öffnen der Pore führen (z.B. $K_{ATP}$-Kanal Öffner).

Die meisten Kaliumkanalöffner, die auf spannungsabhängige Kanäle wirken, fungieren jedoch als "gating modifier". Die Substanzen beeinflussen die Spannungsabhängigkeit der Aktivierung der Kanäle. Durch die Bindung der Substanz verschiebt sich die Aktivierungskurve der Kanäle hin zu negativeren Potentialen. Das hat zur Folge, dass bei gleichem Membranpotential mehr Kanäle geöffnet sind. Retigabine, ein bekannter KCNQ-Kanalöffner wirkt z.B. über diesen Mechanismus.

Die pharmakologische Beeinflussung bestimmter Kaliumkanäle scheint bei einer Vielzahl von Krankheiten von therapeutischem Nutzen zu sein und ist bei einigen Erkrankungen sogar als wirksame Therapie bereits etabliert. Kaliumkanäle sind daher wichtige therapeutische "drug targets". Die Indikationsgebiete sind unter anderem neurologische Erkrankungen, wie Epilepsien (z.B. KCNQ-Kanäle), aber auch Bluthochdruck (z.B. $K_{ATP}$-Kanäle, $K_{Ca}$-Kanäle), Herzrhythmusstörungen (z.B. $K_{ATP}$-Kanäle, hERG-Kanäle, KCNQ-Kanäle, HCN-Kanäle), Schmerzzustände (z.B. KCNQ-Kanäle), Diabetes (z.B. $K_{ATP}$-Kanäle), Asthma, Inkontinenz (z.B. $K_{ATP}$-Kanäle) und andere Erkrankungen. Folglich liegt in der Bereitstellung von Verbindungen, die Ionenkanäle aktivieren oder inaktivieren, ein Potential zur Entwicklung von neuen und hochwirksamen therapeutischen Konzepten.

In den vergangen Jahren konnte eine Reihe neuer Ionenkanäle bzw. Untereinheiten von Ionenkanälen identifiziert werden. Dies hat das Interesse an neuen Wirkstoffen, die die Aktivität solcher Kanäle verändern, stimuliert.

Die Suche nach neuen potenten Wirkstoffen, die Kaliumkanäle beeinflussen, erfordert die Entwicklung von Assays, die es erlauben, mit möglichst hohem Durchsatz, den Einfluss von Substanzen auf die Aktivität des jeweiligen Kaliumkanaltyps zu testen (Übersicht: FORD ET AL., Progress in Drug Research, Vol. 58, S. 133 ff, 2002).

Die Druckschrift US 6,329,367 offenbart Kaliumkanalagonisten, die zur Behandlung verschiedener Krankheiten, u.a. des zentralen Nervensystems oder des Herz-Kreislaufsystems verwendet werden können.

Die Druckschrift WO 02/32419 offenbart Kaliumkanalagonisten und deren Verwendung zu therapeutischen Zwecken.

Der Offenbarungsgehalt der Druckschrift EP 1760158 liegt darin, ein Verfahren zur Bestimmung des Effekts von Testsubstanzen auf das Membranpotential von Mitochondrien zu präsentieren. Dieses Verfahren soll auch zur Identifikation von Kaliumkanalöffnern dienen.

Die Veröffentlichung "Comparative Study of Membrane Potential-Sensitive Fluorescent Probes and Their Use in Ion Channel Screening Assays" von Wolff et al. im Journal of Biomolecular Screening (Bd. 8, Nr. 5, 2003, Larchmont, NY, US) befasst sich mit einer vergleichenden Studie, in der vier unterschiedliche Membranpotential-abhängige Farbstoffsysteme und deren Interaktion mit acht verschiedenen Kanalmodulatoren zum Zwecke der Identifikation neuer Ionenkanäle analysiert wurden.

Derzeit stehen verschiedene Techniken zur Verfügung, um die Aktivität von Kaliumkanälen zu messen: Für die Untersuchung der Aktivität aller Arten von Ionenkanälen gilt die sogenannte Patch-Clamp-Technik als "Gold Standard".

Lange Zeit erschien diese Technik aufgrund des sehr geringen Durchsatzes als ungeeignet für ein Wirkstoffscreening. Um die Patch-Clamp-Technik als HTS-Technologie (High Throughput Screening) nutzbar zu machen, wird die Automatisierung dieses Verfahrens intensiv vorangetrieben. Erste vielversprechende Assays, z.B. für hERG-Kaliumkanäle (*human ether-a-go-go related gene*), sind mit diesen Systemen bereits entwickelt worden. Vor allem für Assays mit spannungsaktivierten Kaliumkanälen, wie die KCNQ-Kanäle, ist diese Technologie sehr vielversprechend. Sie ermöglicht, die Wirkung von Substanzen auf die Kanäle unter physiologischen Bedingungen zu testen. Nicht-invasive, zelluläre Assays erlauben allerdings momentan immer noch eine schnellere Wirkstoffsuche.

Kaliumkanäle leiten neben Kalium immer auch in bestimmtem Maße Rubidium ($Rb^+$). Rubidium-Ionen haben sehr ähnliche Eigenschaften wie Kalium-Ionen, weshalb Kaliumkanäle nur schlecht zwischen diesen beiden Ionen diskriminieren können. Unter physiologischen Bedingungen spielt $Rb^+$ keine Rolle, da es nur in Spuren im Körper vorkommt. Die Leitfähigkeit der Kanäle für Rubidium hat man sich jedoch für die Entwicklung nicht-invasiver Assays zu Nutze gemacht. Für Kaliumkanäle, wie die spannungsabhängigen KCNQ- hERG-, $K_v1.3$,- Kanäle, sowie $K_{ATP}$-Kanäle wurden klassische $Rb^+$-Fluß-Assays im Mikrotiterplattenformat entwickelt (z.B. SCOTT ET AL., Analytical Biochemistry, 319 (2003), 251-257). Die Zellen, die den jeweiligen Kanal exprimieren, werden dafür zunächst mit radioaktivem Rubidium beladen. Dann wird z.B. der Eflux von radioaktivem Rubidium aus den Kulturzellen nach einer gezielten Aktivierung des jeweiligen Kaliumkanals gemessen. Mittlerweile ist es auch möglich, bei solchen Assays nicht-radioaktives Rubidium zu verwenden. Der Rubidium-Eflux wird dann mit Hilfe der Atomabsorptionsspektroskopie nachgewiesen. Der experimentelle Aufwand und die Kosten solcher Assays sind jedoch sehr groß, weshalb sich diese Verfahren bisher nicht durchsetzen konnten. Sowohl die Patch-Clamp-Technik als auch Rubidium-Flux Assays sind prinzipiell dazu geeignet, nach Kaliumkanalöffnern und Blockern zu suchen.

Am weitesten verbreitet sind allerdings zellbasierte Assay-Technologien, bei denen mit Hilfe von speziellen Fluoreszenzfarbstoffen die Wirkung von Substanzen auf die Kaliumkanäle optisch ausgelesen wird (z.B. WHITEAKER ET AL. Journal of Biomolecular Screening, Vol. 6, No. 5, 2001). Dabei verwendet man sog. spannungssensitive Fluoreszenzfarbstoffe. In der Zelle und in der Zellmembran zeigen diese Farbstoffe eine intensive Fluoreszenz, während sie in der wässrigen extrazellulären Phase nur sehr schwach fluoreszieren. Eine Änderung des Membranpotenzials führt bei diesen Farbstoffen zu einer Umverteilung von Farbstoffmolekülen zwischen extrazellulärer Phase, der Zellmembran und dem Zellinneren. Dadurch führt eine Änderung des Membranpotenzials zu einer Änderung der Fluoreszenzintensität der Zelle. Eine Depolarisation des Membranpotenzials führt dabei zu einer Zunahme der Fluoreszenzintensität, während die Fluoreszenzintensität abnimmt, wenn die Zelle hyperpolarisiert. Die absolute Änderung der Fluoreszenzintensität kann dabei ca. 1-2,5% pro 1 mV Änderung des Membranpotenzials betragen, je nach Farbstoff und Zellsystem.

Im Nachfolgenden wird vorgestellt, wie mit Hilfe von spannungssensitiven Fluoreszenzfarbstoffen nach Wirkstoffen gesucht werden kann, die die Aktivität von Kaliumkanälen beeinflussen.

"Direkte" Membranpotentialassays für die Suche nach Kaliumkanalmodulatoren: Mit dieser Art von Assays lassen sich sowohl Kaliumkanalblocker (Depolarisierungsassay) als auch Kaliumkanalöffner (Hyperpolarisierungsassay) identifizieren. Der Einfluss von Wirkstoffen auf das Membranpotenzial von Zellen, die den entsprechenden Kaliumkanal exprimieren, wird hierfür direkt verfolgt. Die Zellen werden dazu zunächst mit dem Fluoreszenzfarbstoff inkubiert, und die Fluoreszenzintensität der Zellen wird anschließend vor und nach Applikation des Wirkstoffs aufgenommen.

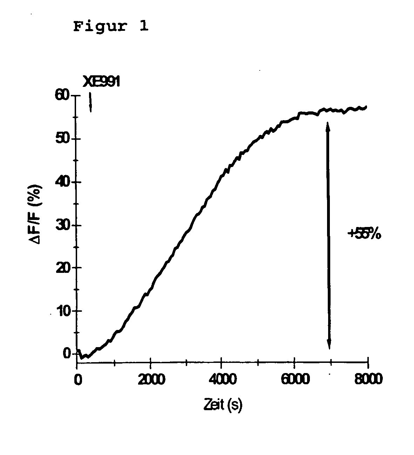

Die Figur 1 zeigt als Beispiel den Zeitverlauf der Fluoreszenzintensität von Zellen, die KCNQ-Kanäle exprimieren, vor und nach der Applikation einer sättigenden Konzentration von XE991, einem bekanntem KCNQ-Kanalblocker.

Nach der Applikation des KCNQ-Kanalblockers beobachtet man eine Zunahme der Fluoreszenzintensität um etwa 55%. Das Blockieren der KCNQ-Kanäle durch XE991 verringert die Permeabilität der Membran für Kaliumionen. Nach vorstehender Gleichung 2 wird dadurch der Einfluss des Kalium-Gleichgewichtspotenzials auf das Membranpotenzial der Zellen reduziert, und es stellt sich ein neues, positiveres Membranpotenzial ein. Diese Depolarisation führt zu der Zunahme der Fluoreszenzintensität der Zellen.

Je nach Kaliumkanaltyp und Zellsystem lässt sich in solchen "Depolarisierungsassays" durch Applikation eines Kaliumkanalblockers eine Zunahme der Fluoreszenzintensität von 100% und mehr beobachten. Bei spannungsabhängigen Kaliumkanälen, wie den KCNQ-Kanälen, sind die Fluoreszenz- bzw. Membranpotenzialänderungen jedoch eher geringer.

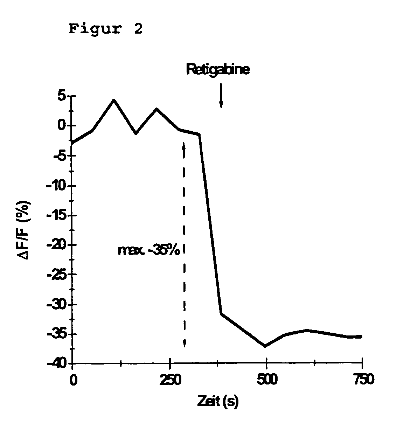

Wirkstoffe, die Kaliumkanäle in der Zellmembran öffnen (bzw. deren Spannungsabhängigkeit der Aktivierung zu negativeren Potentialen verschieben; sog. "gating modifier") führen ebenfalls zu einer Änderung des Membranpotentials. Die Figur 2 zeigt beispielhaft den Zeitverlauf der Fluoreszenzintensität von Zellen, die KCNQ-Kanäle exprimieren, vor und nach der Applikation von Retigabine, einem bekannten KCNQ-Kanalöffner (bzw. "gating modifier"). Die Applikation von Retigabine führt zu einer Abnahme der Fluoreszenzintensität von etwa 35-40%. Die Öffnung von KCNQ-Kanälen durch Retigabine erhöht die Kaliumpermeabilität der Membran. Nach vorstehender Gleichung 2 wird dadurch der Einfluss des Kalium-Gleichgewichtspotenzials auf das Membranpotenzial der Zellen erhöht, und es stellt sich ein neues, negativeres Membranpotenzial ein. Diese Hyperpolarisation führt zu einer Abnahme der Fluoreszenzintensität der Zellen.

Der dynamische Bereich solcher Hyperpolarsierungsassays für die Suche nach Kaliumkanalöffnern (bzw. "gating modifier") ist allerdings meist nicht größer als 50% und somit eher gering. Der Hauptgrund dafür ist, dass das Ausmaß der Hyperpolarisation, die durch Kaliumkanalöffner hervorgerufen werden kann, limitiert ist. Das Membranpotenzial kann höchstens den Wert des Kalium-Gleichgewichtspotenzials annehmen, weshalb das Ausmaß der Hyperpolarisation meist auf 20-30 mV begrenzt ist.

Neben dem eher geringen dynamischen Bereiche haben sowohl die Depolarisationsassays für Kaliumkanalblocker als auch die Hyperpolarisationsassays für Kaliumkanalöffner (oder "gating modifier") weitere Nachteile:

Die verwendeten Fluoreszenzfarbstoffe neigen dazu, mit den Testsubstanzen zu interagieren. Das führt nicht selten zu unspezifischen Fluoreszenzänderungen der Farbstoffe von bis zu 20% und mehr. Diese unspezifischen Fluoreszenzänderungen sind oft nur schwer von den Kanal-spezifischen Signalen zu unterscheiden, was bei einem Hochdurchsatzscreening zu vermehrten falsch-negativen und falsch-positiven Ergebnissen führen kann

Für die Suche nach Kaliumkanalblockem wird daher meist ein alternatives "indirektes" Assay-Verfahren verwendet, sogenannte "ion jump" Assays.

Bei dieser Art von Membranpotenzial-Assays, die bisher ausschließlich für die Suche nach Kaliumkanalblocker verwendet wurde, wird die Wirkung von Substanzen auf die jeweiligen Kaliumkanäle indirekt bestimmt. Die Zellen, die den jeweiligen Kaliumkanal exprimieren, werden hierfür zunächst mit einem spannungssensitiven Fluoreszenzfarbstoff inkubiert. In einem zweiten Schritt werden dann die Wirkstoffe appliziert. Anschließend wird die Fluoreszenzintensität der Zellen vor und nach einer Applikation von Kaliumionen zum extrazellulären Medium verfolgt. Die extrazelluläre Kaliumionenkonzentration wird durch die $K^+$-Applikation von 1-5 mM auf 50-100 mM erhöht.

Die Erhöhung der extrazellulären Kaliumkonzentration führt dazu, dass das Gleichgewichtspotenzial für Kalium positiver wird (Gleichung 1). Da das Membranpotenzial der Zellen vom Gleichgewichtspotenzial für Kalium mitbestimmt wird, depolarisieren die Zellen infolge der $K^+$-Applikation. Das Ausmaß der Depolarisation hängt allerdings davon ab, wie groß die Permeabilität der Membran für Kalium ist (Gleichung 2). Je geringer die Permeabilität für Kalium, desto kleiner ist die Depolarisation und desto kleiner ist die Fluoreszenzänderung der Zellen infolge der $K^+$-Applikation. Die Permeabilität der Membran für Kalium nimmt ab, wenn die Kaliumkanäle blockiert werden. Folglich ist in Anwesenheit eines Blockers die Fluoreszenzzunahme der Zellen nach $K^+$-Applikation kleiner, verglichen mit der Fluoreszenzzunahme in Abwesenheit eines Blockers.

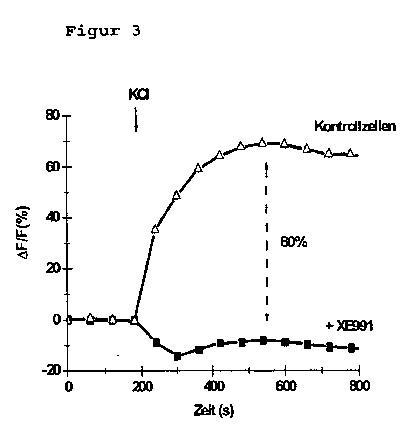

Die Figur 3 zeigt als Beispiel die Fluoreszenzantwort von Zellen, die KCNQ-Kanäle exprimieren, auf die Injektion von Kalium zum extrazellulären Medium in Ab- und Anwesenheit eines KCNQ-Kanalblockers. In Abwesenheit eines KCNQ-Kanalblockers (Kontrollzellen) führt die Kalium-Applikation zu einer Zunahme der Fluoreszenzintensität um etwa 80%. Im Gegensatz dazu beobachtet man in Anwesenheit einer sättigenden Konzentration des bekannten KCNQ-Kanalblockers XE991 keine Fluoreszenzzunahme nach der Kalium-Applikation. Die Differenz der Amplituden der Fluoreszenzantworten in Ab- und Anwesenheit eines KCNQ-Kanalblockers, der sogenannte dynamische Bereich des Assays, beträgt in diesem Fall etwa 80%.

Bei solchen "ion jump" Assays können sich, je nach Kaliumkanaltyp und Zellsystem, die Amplituden der $K^+$-induzierten Fluoreszenzänderung in An- und Abwesenheit eines Blockers um bis zu 300% unterscheiden. Aufgrund dieses meist großen dynamischen Bereiches, der großen Fluoreszenzsignale und guten Reproduzierbarkeit, finden "ion jump =Assays seit Jahren weit verbreitet Anwendung bei der Suche nach Kaliumkanalblockem (z.B. FALCONER ET AL.,

Journal of Biomolecular Screening, Vol. 7, No. 5, 2002; WOLFF ET AL., Journal of Biomolecular Screening 8(5), 2003). Sie wurden bisher nicht für die Suche nach Kaliumkanalöffnern verwendet. Auch über den Effekt von Kaliumkanalöffnern in "ion jump"-Assays war bisher nichts bekannt.

Bei den vorstehend beschriebenen "ion jump" Assays handelt es sich um robuste und etablierte Assays zur Auffindung von Kaliumkanalblockern, d.h., Verbindungen, die Kaliumkanäle blockieren oder schließen.

Im Gegensatz dazu stehen zur Auffindung von Verbindungen, die Ionenkanäle und insbesondere Kaliumkanäle öffnen (sogenannte Kanalöffner oder auch Agonisten oder auch "gating modifier"), nur die meist wenig zufriedenstellenden und unzuverlässigen Hyperpolarisationsassays zur Verfügung. Im Gegensatz zu den zuvor beschriebenen robusten und etablierten "ion jump" Blocker-Assays, sind die bisher bekannten Membranpotenzial-Assays für die Suche nach Kaliumkanalöffnern, insbesondere für spannungsabhängige Kaliumkanäle wie die KCNQ-Kanäle, deutlich limitiert. Der dynamischen Bereich, die Zuverlässigkeit und Reproduzierbarkeit sind in der Regel gering. Zur Suche nach Kanalöffnern werden bisher ausschließlich Hyperpolarisationsassays verwendet (z.B. WHITEAKER ET AL., Journal of Biomolecular Screening, Vol. 6, No. 5, 2001; GOPALAKRISHNAN ET AL., The Journal of Pharmacology and Experimental Therapeutics, Vol. 303, No. 1 (2002), pp. 379-386 and British Journal of Pharmacology (2004) 143, 81-90).

Diese Assays liefern vor allem bei spannungsabhängigen Kaliumkanälen nur sehr kleine Messfenster (kleiner dynamischer Bereich). Das geringe Messfenster ist durch die biophysikalischen Eigenschaften der Kanäle bedingt: Kulturzellen wie z.B. CHO- oder HEK-Zellen haben in der Regel ein Membranpotential von etwa - 20 bis -40 mV, weit entfernt vom Kaliumgleichgewichtspotential. Der Grund dafür ist, dass diese Zellen kaum eigene Kalium-Leitfähigkeiten exprimieren. Folglich wird das Membranpotential dieser Zellen nur wenig vom Kaliumgleichgewichtspotential dominiert.

Durch die heterologe Expression eines spannungsabhängigen Kaliumkanals verändert sich das Membranpotential der Kulturzellen. Die Basalaktivität der spannungsabhängigen Kaliumkanäle führt dazu, dass sich das Membranpotential in Richtung des Kaliumgleichgewichtspotentials verschiebt - das Membranpotential wird negativer. Diese Hyperpolarisation ist allerdings selbstlimitierend, da die Kanäle wiederum durch die Hyperpolarisation geschlossen werden. Folglich stellt sich ein neuer Gleichgewichtszustand ein. Bei CHO- und HEK-Zellen führt die Expression eines spannungsabhängigen Kaliumkanals dazu, dass sich ein Membranpotential von etwa -60 bis -70 mV einstellt.

Werden nun die Kanäle durch einen Kaliumkanalöffner aktiviert, so verschiebt sich das Membranpotential noch weiter in Richtung des Kaliumgleichgewichtspotentials, das bei etwa -90 mV liegt. Dabei kann das Membranpotential nicht negativer als das Kaliumgleichgewichtspotential werden. Da die Zellen durch die Basalaktivität der Kaliumkanäle bereits ein Membranpotential von etwa -60 bis -70mV besitzen, ist die Amplitude der Kanalöffner-induzierten Hyperpolarisation auf 20 bis 30 mV beschränkt.

Da bei den bisher verwendeten Hyperpolarisationsassays für spannungsabhängige Kaliumkanäle genau diese Kanalöffner-induzierte Hyperpolarisation mit Hilfe von spannungssensitiven Fluoreszenzfarbstoffen direkt verfolgt wird, ist dementsprechend die Amplitude der Fluoreszenzantworten ebenfalls limitiert. Deshalb ist der dynamische Bereich der bisher verwendeten Hyperpolarisationsassays für spannungsabhängige Kaliumkanäle gering, was eine verlässliche Struktur-Wirkungs-Analyse bisher erschwerte.

Bei den nicht spannungsabhängigen Kanälen wie z.B. $K_{ATP}$-Kanäle oder auch einige Kalzium-aktivierte Kaliumkanäle ist der dynamische Bereich der Hyperpolarisationsassays meist etwas größer, da diese Kanäle in Abwesenheit eines Kanalöffners eine geringere Basalaktivität haben und somit das Membranpotential der Zellen in Abwesenheit eines Kanalöffners positiver ist.

Der kleine dynamische Bereich der Hyperpolarisationsassays für Kaliumkanalöffner und auch die häufigen unspezifischen Farbstoff-Wirkstoff Interaktionen können eine verlässliche Struktur-Wirkungs-Analyse von Wirkstoffen verhindern bzw. führen zu eher unbefriedigenden Ergebnissen. Es besteht somit ein Bedarf nach Assays für Kaliumkanalöffner, insbesondere Öffner von spannungsabhängigen Kaliumkanälen.

Der Erfindung liegt somit die Aufgabe zugrunde, ein Verfahren bereitzustellen, mit dem Kaliumkanalöffner einfach und zuverlässig ermittelt werden können. Das Verfahren soll sich durch Schnelligkeit, Effizienz und eine hohe Empfindlichkeit auszeichnen und soll universell für die Testung beliebiger spannungsabhängiger Kaliumkanäle auf diese öffnende Verbindungen geeignet sein. Weiterhin soll das Verfahren geeignet sein, die Eignung von beliebigen Verbindungen, einen spannungsabhängigen Kaliumkanal zu öffnen, zu testen. Ferner soll das Verfahren eine niedrige falschpositive Trefferquote haben, zu wenig falsch-negativen Ergebnissen führen und sich durch eine hohe Reproduzierbarkeit auszeichnen. Ferner soll sich das Verfahren sowohl für einen Einsatz im Labormaßstab als auch für ein Hochdurchsatzscreening (High Troughput Screening, HTS) und zur Automatisierung eignen. Darüber hinaus soll das Verfahren kostengünstig sein und zu keiner unnötigen Belastung der Umwelt führen, z.B. durch radioaktive oder toxische Chemikalien. Bevorzugt soll sich das Verfahren für die Suche nach Öffnern spannungsabhängiger Kaliumkanäle eignen.

Überraschenderweise wurde nun gefunden, dass sich das Messprinzip von "ion jump"-Assays erfolgreich und sehr empfindlich zur Identifizierung von Verbindungen, die Kaliumkanäle öffnen bzw. von "gating modifiern", die die Spannungsabhängigkeit von spannungsaktivierten Kaliumkanälen beeinflussen, verwenden lässt. Ein "gating modifier" wirkt als Kaliumkanalöffner, wenn die Spannungsabhängigkeit der Kaliumkanäle bzw. die halbmaximale Aktivierungsspannung zu negativeren Potenzialen verschoben wird. Ferner wurde gefunden, dass durch eine Modifizierung des "ion jump"-Messprinzips ein neuer Assay etabliert werden konnte, mit dem einfach und wirksam die agonistische, d.h. die öffnende bzw. die Spannungsabhängigkeit der Aktivierung modifizierende Aktivität einer Zielverbindung auf einem Kaliumkanal ermittelt werden kann. Es wurde gefunden, dass sich dieses Assayformat mit hoher Empfindlichkeit zur gezielten Identifizierung und Charakterisierung von Kaliumkanalöffnern eignet. Es wurde ferner gefunden, dass sich das Messprinzip von "ion jump"-Assays besonders zur Identifizierung von Verbindungen, die spannungsabhängige Kaliumkanäle öffnen, eignet.

Die Erfindung betrifft somit ein Verfahren zur Identifizierung der agonistischen Aktivität einer Zielverbindung auf einen spannungsabhängigen Kaliumkanal, das dadurch gekennzeichnet ist, dass man a) eine Population von Zellen, die einen Kaliumkanal exprimieren, bereitstellt, b) die Zellen gemäß a) mit einem spannungssensitiven Fluoreszenzfarbstoff inkubiert, c) die Zielverbindung dem Reaktionsansatz von b) zusetzt, d) einen Wert $F_1$ der Fluoreszenzintensität der Zellen bestimmt, e) Kaliumionen in einer physiologisch verträglichen Konzentration zusetzt, f) einen Wert $F_2$ der Fluoreszenzintensität der Zellen bestimmt, und g) die Fluoreszenzintensität F2 mit der Fluoreszenzintensität F1 vergleicht und daraus die agonistische Aktivität der Zielverbindung auf den Kaliumkanal ermittelt. F2 und F1 werden dafür bevorzugt wie folgt verrechnet:

$$\left(\frac{F_2 - F_1}{F_1}\right) \times 100 = \frac{\Delta F}{F}\,(\%)$$

Um zu ermitteln, ob eine Substanz eine agonistische Aktivität besitzt, kann z.B. mit $\frac{\Delta F}{F}$ mit $\left(\frac{\Delta F}{F}\right)_K$,

von Kontrollzellen verglichen werden. $\left(\frac{\Delta F}{F}\right)_K$ wird ermittelt, indem man a) eine Population von Zellen, die den Kaliumkanal exprimieren, mit einem Fluoreszenzfarbstoff inkubiert, b) dem Reaktionsansatz von a) anstatt einer Zielverbindung lediglich einfache Pufferlösung zusetzt, c) einen Wert $F_{1K}$ der Fluoreszenzintensität der Kontrollzellen bestimmt, d) Kaliumionen in einer physiologisch verträglichen Konzentration zusetzt, e) einen Wert $F_{2K}$ der Fluoreszenzintensität der Kontrollzellen bestimmt, und f) $F_{2K}$ und $F_{1K}$ bevorzugt wie folgt verrechnet:

$$\left(\frac{F_{2K} - F_{1K}}{F_{1K}}\right) \times 100 = \left(\frac{\Delta F}{F}\right)_K (\%)$$

Eine Substanz besitzt eine agonistische Aktivität auf den Kaliumkanal, wenn $\frac{\Delta F}{F}$ größer als $\left(\frac{\Delta F}{F}\right)_K$ ist:

$$\frac{\Delta F}{F} \;>\; \left(\frac{\Delta F}{F}\right)_K$$

Unabhängig von dem Vergleich von $\frac{\Delta F}{F}$ mit $\left(\frac{\Delta F}{F}\right)_K$ lässt sich auch auf eine agonistische Aktivität einer Zielverbindung schließen, wenn mit steigender Dosierung der Zielverbindung eine Zunahme von $\frac{\Delta F}{F}$ zu beobachten ist.

Unter agonistischer Aktivität einer Zielverbindung auf einen Kaliumkanal soll jede diesen Kanal öffnende Aktivität der Zielverbindung verstanden werden. Eine öffnende Aktivität liegt vor, wenn die Spannungsabhängigkeit der Kanäle bzw. die halbmaximale Aktivierungsspannung zu negativeren Potenzialen verschoben wird. Somit umfasst dieser Begriff auch die sogenannten "gating modifier". Mit dem erfindungsgemäßen Verfahren können alle Arten von einen Kaliumkanal-öffnenden Verbindungen ermittelt werden, unabhängig davon, ob diese Verbindungen den Kanal vollständig oder nur in einem gewissen Ausmaß öffnen.

Überraschend ist, dass dieses bisher für Ionenkanalöffner und insbesondere Kaliumkanalöffner unbekannte Assay-Prinzip den bekannten Hyperpolarisationsassays weit überlegen ist. Der dynamische Bereich dieses neuartigen "ion jump" Assays beträgt für KCNQ-Kanäle etwa 250% und ist damit mehr als sechsmal größer ist als der dynamische Bereich eines Hyperpolarisationsassays, der für KCNQ-Kanäle lediglich etwa 35-40% beträgt.

Dieser innovative Assay für Kaliumkanalöffner ist extrem zuverlässig, reproduzierbar und erlaubt eine verlässliche Struktur-Wirkungs-Analyse. Das erfindungsgemäße Assay-Format für Kaliumkanalöffner ist somit den bisher verwendeten Hyperpolarisationsassayformaten überlegen.

Der erfindungsgemäße Assay hat weiterhin den Vorteil, dass bei dem "ion jump" Prinzip nicht die limitierte Kanalöffner-induzierte Hyperpolarisation verfolgt wird. Bei dem "ion jump"-Assays wird vielmehr die Aktivität der Kaliumkanäle indirekt über die Kaliumabhängigkeit des Membranpotentials bestimmt. Die Limitationen der bisher verwendeten Hyperpolarisationsassays kommen hier nicht zum tragen. Deshalb ist der dynamische Bereich des neuen "ion jump"-Assay-Formates sehr viel größer als der herkömmlicher Assays, was eine verlässlichere und sensitivere Wirkstoffsuche vor allem für Öffner von spannungssensitiven Kaliumkanälen ermöglicht. Deshalb ist der neu entwickelte Assay insbesondere bei der Suche nach Öffnern für spannungsabhängige Kaliumkanäle, den bisher verwendeten Assay-Formaten überlegen.

Der sehr große dynamische Bereich des neuen Assay-Verfahrens ermöglicht außerdem, insbesondere Öffner spannungssensitiver Kaliumkanäle, zu identifizieren, die nur eine schwache agonistische Wirkung auf die jeweiligen Kaliumkanäle haben. Solche Wirkstoffe lassen sich mit den bisher verwendeten Hyperpolarisationsassays nur sehr schlecht identifizieren, da der dynamische Bereich dieser Assays meist sehr gering ist.

Da bei dem neuartigen Assay-Verfahren zur Suche nach Kaliumkanalöffnern die Wirkung von Substanzen auf die spannungsabhängigen Kaliumkanäle zudem indirekt anhand der Kalium-induzierten Depolarisation ausgelesen wird, sind unspezifische Farbstoff-Wirkstoff-Interaktionen zu vernachlässigen, wobei grundsätzlich das Prinzip und die Vorteile der spannungssensitiven Farbstoffe ausgenutzt werden können. Das reduziert im Vergleich mit den herkömmlichen Hyperpolarisationsassays das Auftreten von falsch-positiven und falsch-negativen Ergebnissen deutlich und führt zu einer höheren Empfindlichkeit des erfindungsgemäßen Assay-Formats.

Bei dem erfindungsgemäßen Verfahren werden aus "ion jump"-Assays bereits bekannten Verfahrensschritte in an sich bekannter Weise verwendet. Die nachstehend dargelegten Modifikationen des "ion jump"-Assays können bevorzugt vorgenommen werden, um die Eignung und Empfindlichkeit des erfindungsgemäßen Verfahrens zur Identifizierung von Verbindungen, die einen spannungsabhängigen Kaliumkanal öffnen, zu verbessern bzw. zu erhöhen.

Erfindungsgemäß wird zunächst eine Population von Zellen, die den spannungsabhängigen Kaliumkanal exprimieren, mit einem spannungssensitiven Fluoreszenzfarbstoff inkubiert. Es können beliebige Zellpopulationen verwendet werden, solange sie den jeweiligen zu testenden Ionenkanal exprimieren. Solche Zelllinien sind einem Fachmann gut bekannt. Beispiele hierfür sind transient transfizierte Zelllinien, stabile Zelllinien, primäre Zellkulturen und Gewebszellen sowie Zelllinien, die endogen einen Kaliumkanal exprimieren. Beispiele für letztere sind F-11-oder PC-12-Zellen, die endogen KCNQ-Kanäle exprimieren.

Die Züchtung der Zellen erfolgt in an sich bekannter Weise in bekannten Nährmedien. Die Züchtung der Zellen erfolgt wie es auf dem Fachgebiet für die den jeweiligen Kaliumkanal exprimierenden Zellen bekannt ist. Beispielsweise werden KCNQ-Kanäle exprimierende CHO-Zellen (chinese hamster ovary cells) in Rollflaschen als Suspensionskultur oder adhärent in Petrischalen, bevorzugt in Zellkulturflaschen, kultiviert. Als Nährmedium wird ein Minimum Essential Medium (MEM) mit einem Zusatz von Fetal Calf Serum bevorzugt verwendet. Besonders bevorzugt kultiviert man die Zellen in einem Minimum Essential Medium (MEM) $\alpha$ Medium 22571 1x flüssig (Invitrogen,) mit 10 % Fetal Calf Serum (FCS) (Gibco, heat inactivated) bei 37°C, 5 % $CO_2$ und 95 % Luftfeuchtigkeit. Für die Passagierung werden die Zellen mechanisch oder enzymatisch abgelöst, bevorzugt enzymatisch durch Trypsin, ganz besonders bevorzugt enzymatisch durch Accutase (Fa. PAA). Das Umsetzen der Zellen erfolgt in dem Rhythmus, dass bei Aussaat auf Messplatten ein ca. 70%-Konfluenzstadium erreicht war oder bevorzugt nach einem festen Split-Regime.

Anschließend werden die Zellen in Messplatten ausgesät. Zum Abernten aus den Zellkulturflaschen werden die Zellen mit einem geeigneten Puffer gewaschen und mechanisch oder enzymatisch von dem Trägermaterial abgelöst. Bevorzugt verwendet man eine enzymatisch/chemische Methode mit Trypsin und Ethylendiamintetraessigsäure (EDTA). Besonders geeignet ist es, die Zellen bei 70-90%iger Konfluenz mit einem Puffer einer Zusammensetzung von 0,8-2 mM $CaCl_2$, 1-5 mM KCl, 1-2 mM $KH_2PO_4$ 0,2-1 mM $MgCl_2$, 100-200 mM NaCl, 5-10 mM $Na_2HPO_4$, 0-10 mM D-Glucose und 0-0,5 mM Natriumpyruvat-Puffer zu waschen und die Zellen mit einer geringen Konzentration an Trypsin zum Anlösen für 2 bis 15 min bei 37°C zu inkubieren. Ganz besonders geeignet ist es, die Zellen bei 80%iger Konfluenz mit einem Puffer einer Zusammensetzung von 0,9 mM $CaCl_2$, 2,7 mM KCl, 1,5 mM $KH_2PO_4$, 0,5 mM $MgCl_2$, 140 mM NaCl und 8 mM $Na_2HPO_4$ zu waschen und die Zellen zum Anlösen mit 2 ml Accutase (PAA) für 15 min bei 37°C zu inkubieren.

Die abgelösten Zellen werden in einer geeigneten Menge Nährmedium, bevorzugt zwischen 5 und 10 ml, resuspendiert. Die Bestimmung der Zellzahl erfolgt in einer Zählkammer oder in einem Zell-Counter, bevorzugt in einem CASY Zell-Counter (Schärfe System). Die Aussaat erfolgt auf für Fluoreszenzmessungen geeignete 1536-, 384- oder 96-well Messplatten, bevorzugt auf Platten, deren Kunststoff eine bessere Adhärenz der Zellen ermöglicht oder die zu diesem Zweck speziell beschichtet sind. Bevorzugt verwendet man 96-well-Platten mit Poly-D-Lysin-Beschichtung, Poly-L-Lysin-Beschichtung, Collagen-Beschichtung oder Fibronektin-Beschichtung. Ganz besonders bevorzugt sind Corning® CellBIND® 96-well-Platten (Black with clear bottom). Es werden zwischen 10.000 und 40.000 Zellen pro Kavität in den 96-well-Platten ausgesät. Bevorzugt sät man 15.000 bis 25.000 Zellen pro well aus. Ganz besonders bevorzugt werden 20.000 Zellen in 100 μl Nährmedium ausgesät und 16-32 h, bevorzugt 24 h bei 37°C, 5 % $CO_2$ und 95 % Luftfeuchtigkeit inkubiert. Ganz besonders vorteilhaft ist es, die Zellen vor der Inkubation bei 37°C 1 h bei Raumtemperatur zu inkubieren, um eine gleichmäßige Adhärenz der Zellen zu gewähren.

Für andere Kaliumkanäle exprimierende Zellen können andere Zellkulturprotokolle geeignet sein, die jedoch einem Fachmann bekannt sind.

Alternativ können auch Zellen verwendet werden, die sowohl einen Kaliumkanal als auch einen Protein-basierten fluoreszenzoptischen Spannungssensor exprimieren. Solche Zellen sind einem Fachmann gut bekannt und besitzen den Vorteil, dass der Schritt der Inkubation mit einem spannungssensitiven Fluoreszenzfarbstoff wegfallen kann. Die Aufbereitung dieser Zellen für das erfindungsgemäße Verfahren kann zum Beispiel anhand des vorstehend beschriebenen Protokolls für Zellen, die einen Kaliumkanal exprimieren, vorgenommen werden.

Anschließend werden die Zellen gegebenenfalls mit einem spannungssensitiven Fluoreszenzfarbstoff inkubiert bzw. beladen.

Es können verschiedene spannungssensitive Fluoreszenzfarbstoffe zur Inkubation der Zellen verwendet werden. Derartige Farbstoffe sind einem Fachmann gut bekannt und nachstehend beispielhaft aufgeführt.

Spannungssensitive Fluoreszenzfarbstoffe lassen sich prinzipiell in zwei Klassen unterteilen: "langsame" und "schnelle" Farbstoffe. Zu den "langsamen" spannungssensitiven Farbstoffen gehören z.B. positiv geladene Farbstoffe auf Cyanin- bzw. Carbocyanin- oder Rhodaminbasis, oder auch anionische Oxonol-Farbstoffe, Bisoxonolfarbstoffe oder Merocyaninfarbstoffe [z.B. Membrane Potential Kit, $DiS-BAC_2(3)$, $DiBAC_4(3)$, $DiOC_2(3)$; $DisC_3(3)$ $DisC_3(5)$]. Bei dem Produkt Membrane Potential Kit handelt es sich um ein Handelsprodukt der Firma Molecular Devices Corporation. Dieses Kit wird derzeit in zwei Varianten angeboten:

Variante blue: Membrane Potential Assay Kit Blue
Variante red: Membrane Potential Assay Kit Red

In der Literatur wird dieses Kit auch als "FLIPR Membrane Potential Dye" bezeichnet. Beide Varianten des Kits eignen sich zur Durchführung des erfindungsgemäßen Assayverfahrens.

In der Zelle und in der Zellmembran zeigen diese Farbstoffe eine intensive Fluoreszenz, während sie in der wässrigen extrazellulären Phase nur sehr schwach fluoreszieren. Eine Änderung der Membranspannung ($V_m$) führt bei diesen sogenannten Nernst-Farbstoffen zu einer Umverteilung von Farbstoffmolekülen zwischen extrazellulärer Phase, der Zellmembran und dem Zellinneren. Dadurch lässt sich eine spannungsabhängige Änderung der Fluoreszenzintensität der Zelle beobachten. Da der Umverteilungsprozess relativ langsam ist, betragen die Zeitkonstanten für die Änderungen der Fluoreszenzintensität je nach Farbstoff ca. 1-20 Sekunden. Die absolute Änderung der Fluoreszenzintensität beträgt bei dieser Farbstoff-Gruppe ca. 1-2.5% pro 1 mV Änderung des Membranpotenzials.

Zur Klasse der "schnellen" spannungssensitiven Farbstoffe zählen z.B. Styryl-Farbstoffe und auch einige Oxonole (z.B. di-8-ANEPPS, di-4-ANEPPS, RH-421, RH-237 etc.) und auch Hemicyanine (z.B. Annine-3 und Annine-6). Diese amphiphatischen Moleküle fluoreszieren praktisch nicht in wässriger Lösung. Sie lagern sich jedoch in die Membran von Zellen ein und zeigen dort eine starke Fluoreszenz. Eine Änderung von $V_m$ führt bei den sog. elektrochromen Farbstoffen (z.B. Styryl-Farbstoffe) zu einer Änderung der Elektronenverteilung im Farbstoffmolekül (sog. "stark shift"). Dadurch verschiebt sich das Fluoreszenzspektrum der Farbstoffe. Andere "schnelle" Farbstoffe (z.B. einige Oxonole) verändern spannungsabhängig ihre Position in der Membran, was ebenfalls zu einer Änderung der spektralen Eigenschaften führt. Die Zeitkonstanten für diese Prozesse betragen weniger als eine Millisekunde. Die spannungsabhängige Verschiebung des Spektrums ist sehr gering. Deshalb kann selbst bei optimaler Filterwahl meist nur eine geringe (ca. 0,1 % pro 1 mV) Änderungen der Fluoreszenzintensität detektiert werden.

Für HTS-Assays sind darüber hinaus auch innovative Farbstoff-Kombinationen erfindungsgemäß interessant, bei denen spannungsabhängig Förster-Resonanz-Energietransfer (FRET) stattfindet. Seit einiger Zeit sind solche Farbstoffsysteme zur Detektion des Membranpotenzials bei den Firmen Invitrogen und Axiom erhältlich. Da diese FRET-Systeme auf "langsamen" Farbstoffen basieren liegt die Empfindlichkeit in der Größenordnung der langsamen Farbstoffe.

Neben den hier beschriebenen Farbstoffen gibt es auch Beispiele für Proteinbasierte Spannungssensoren. z.B. die sogenannten Flash, Flare, SPARC und VSFP-1 Proteine. Solche "fluorescent protein voltage sensitive probes (FPvoltage sensors)" könnten in Zukunft die bisher verwendeten Farbstoffe ablösen. Solche Spannungssensoren sind für das erfindungsgemäße Verfahren prinzipiell geeignet. Solche Protein-basierten fluoreszenzoptischen Spannungssensoren werden von der Zelle, die den Kaliumkanal exprimiert, ebenfalls exprimiert.

Für das erfindungsgemäße Assay-System eigenen sich insbesondere Farbstoffe aus der Gruppe der "schnellen" spannungssensitiven Farbstoffe, wie z.B. Styryl-Farbstoffe (ANEPPS-Farbstoffe), Oxonol-Farbstoffe (z.B. RH421) und Hemicyanin-farbstoffe (ANINNE-Farbstoffe). Auch besonders geeignet für das erfindungsgemäße Assay-Format sind proteinbasierte Spannungssensoren wie z.B. Flash, Flare, SPARC und VSFP-1 Proteine, mit denen die Zellen, die den jeweiligen Kaliumkanal exprimieren, transient oder stabil transfiziert werden.

Bevorzugt werden für den erfindungsgemäßen Assay FRET-basierte Farbstoff-kombinationen verwendet, die auf "langsamen" Farbstoffen basieren, wie sie z.B. von den Firmen Invitrogen oder Axiom angeboten werden. Ein FRET-basiertes Farbstoffsystem der Firma Invitrogen ist als "Voltage Sensor Probes" ("VSP") im Handel erhältlich. Es enthält die Farbstoffe $DisBAC_2(3)$ oder $DisBAC_4(3)$ sowie das membrangebundene Coumarin-Phospholipid "CC2-DMPE". Ebenfalls besonders geeignet ist das FRET-basierte Farbstoffsystem der Firma Axiom Bioscience. Es enthält die Farbstoffkombination $DisBAC_1(3)/DisBAC_1(5)$. Auch andere FRET-Farbstoffsysteme sind für das Assay-System geeignet.

Besonders bevorzugt werden Farbstoffe aus der Gruppe der "langsamen" spannungssensitiven Fluoreszenzfarbstoffe verwendet, wie z.B. Membrane Potential Kit oder $DiSBAC_2(3)$-, $DiBAC_4(3)$-, $DiOC_2(3)$-, $DisC_3(3)$-, $DisC_3(5)$-Farbstoffe.

Bevorzugt werden in dem erfindungsgemäßen Assay-Format Membranpotentialänderungen anhand von Änderungen der Fluoreszenzintensität von spannungssensitiven Farbstoffen ausgelesen. Es ist aber auch möglich, dass man anstatt der Fluoreszenzintensität eine spannungsabhängige Änderung der Fluoreszenzlebenszeit von Farbstoffen oder fluoreszierenden Proteinen als Messparameter verwendet.

Es ist einem Fachmann bekannt, in welcher Weise Kaliumkanal-exprimierende Zellen in geeigneter Weise mit einem spannungssensitiven Fluoreszenzfarbstoff inkubiert werden. Inkubationszeit, Inkubationsbedingungen sowie Mengenverhältnisse Zellen/Fluoreszenzfarbstoff hängen stark von dem jeweils verwendeten Farbstoff ab und können von einem Fachmann durch Routineversuche leicht ermittelt werden.

Die nachstehenden Ausführungen sind beispielhaft für bevorzugte Ausführungsformen der Erfindung.

Um beispielsweise KCNQ-Kanäle-exprimierende CHO-Zellen mit einem spannungssensitiven Farbstoff zu beladen, wird zunächst das Nährmedium, in dem die Zellen gezüchtet wurden, abgenommen.

Die Zellen werden dann mit einem geeigneten Puffer gewaschen. Anschließend überschichtet man die Zellen mit dem in Puffer gelöstem Farbstoff und inkubiert sie zwischen 2 min und 1,5 h. Der Farbstoff wird dabei aus den vorstehenden Farbstoffen ausgewählt. Bevorzugt sollte man die Zellen 30 bis 45 min mit dem Farbstoff inkubieren. Ganz besonders gute Ergebnisse erzielt man, wenn man die Zellen 45 min inkubiert.

Als Puffer eignet sich eine Lösung einer Zusammensetzung von 0,8-4 mM $CaCl_2$, 0-100 mM HEPES, 0,5-10 mM KCl, 0-2 mM $KH_2PO_4$, 0,4-4 mM $MgCl_2$, 0-1 mM $MgSO_4$, 100-200 mM NaCl, 0-40 mM $NaHCO_3$, 0-10 mM $Na_2HPO_4$, 0-20 mM D-Glucose, 0-0,05 mM Phenolrot und 0-0,5 mM Natriumpyruvat. Besonders geeignet ist ein Puffer einer Zusammensetzung von 1-2 mM $CaCl_2$, 0,5-6 mM KCl, 0,5-2 mM $MgCl_2$, 0,2-0,5 mM $MgSO_4$, 100-150 mM NaCl, 2-5 mM $NaHCO_3$, 0,2-0,5 mM $Na_2HPO_4$, 5-10 mM D-Glucose und 5-15 mM HEPES.

Ganz besonders vorteilhaft ist es wenn man die Zellen mit 200 $\mu$l extrazellulärer Lösung (ES; 120 mM NaCl, 1 mM KCl, 10 mM HEPES, 2 mM $CaCl_2$, 2 mM $MgCl_2$, 10 mM Glucose; pH 7,4) wäscht und anschließend mit 100 $\mu$l einer Farbstofflösung (1 Vial Membrane Potential Assay Kit, Red gelöst in 200 ml ES-Lösung) 45 min bei Raumtemperatur inkubiert.

Das vorstehende beispielhafte Protokoll zur Inkubation von Kaliumkanalexprimierenden Zellen mit einem Fluoreszenzfarbstoff kann in Abhängigkeit von den verwendeten Zellen und dem verwendeten Farbstoff von einem Fachmann in an sich bekannter Weise bzw. durch einfache Routineversuche ermittelbar angepasst werden.

Anschließend wird die zu testende Zielverbindung dem Reaktionsansatz zugesetzt. Die Zielverbindung wird dabei als Lösung in einem geeigneten Lösungsmittel zugesetzt. Die Menge der Zielverbindung richtet sich nach dem zu testenden Kaliumionenkanal.

Nach der Farbstoffinkubation wird die Testsubstanz in einem geeigneten Puffer mit einem physiologisch verträglichen Anteil an Lösungsmittel/Vehikel zugegeben und auf den Zellen circa 1 min bis 2,5 h inkubiert. Die Inkubationszeit hängt von der Art des zu testenden Kaliumionenkanals ab. Bevorzugte Inkubationszeiten liegen zwischen 15 min und 1,5 h. Besonders bevorzugt inkubiert man die Zellen mit der Substanz für 30 min. Die Konzentration der zugesetzten Kaliumionen liegt zwischen 10 mM bis 150 mM (Endkonzentration KCl-Lösung), bevorzugt 80 mM bis 120 mM, ganz besonders bevorzugt zwischen 90 mM und 100 mM KCl.

Vor Zugabe der Kaliumionen wird ein Wert $F_1$ der Fluoreszenzintensität der Zellen bestimmt. Die Bestimmung der Fluoreszenzintensität erfolgt in auf dem Fachgebiet an sich bekannter Weise, z.B. mit einer CCD-Kamera oder einem Photomultiplier.

Die Messungen können mit jedem handelsüblichen Fluoreszenzreader durchgeführt werden, besonders geeignet erwiesen sich BMG-Typ Fluostar oder BMG-Typ Polarstar oder Molecular Devices-Flex. Die Fluoreszenz wird bei 525 nm angeregt und bei 560 nm detektiert.

Anschließend wird erneut die Fluoreszenzintensität der Zellen bestimmt.

Die Erhebung des Fluoreszenzwerts $F_2$ erfolgt zu einem gleichbleibenden Zeitpunkt nach KCl-Depolarisation, bevorzugt zum Zeitpunkt des Maximums (Topvalue) der KCl-Depolarisation. Der $\frac{\Delta F}{F}$-Wert der zugehörigen Testsubstanz wird unter Verwendung des F2-Werts im Substanzwell und dem Fluoreszenzwert zu einem festgelegten und für alle Substanzen identischen Zeitpunkt der Basislinie des Substanzwells ($F_1$) ermittelt:

$$\left(\frac{F_2 - F_1}{F_1}\right) \times 100 = \frac{\Delta F}{F}(\%)$$

Von diesem substanzspezifischen $\frac{\Delta F}{F}$-Wert wird der Mittelwert der $\left(\frac{\Delta F}{F}\right)_K$-Werte der Vehikelkontrollen subtrahiert und somit der substanzspezifische dynamische Bereich (mit der Einheit Prozent) erfasst. Dieser Wert kann dann prozentual als Anteil am Effekt (dynamischer Bereich) einer für das Zielprotein relevanten Referenzsubstanz angegeben werden.

Eine Substanz besitzt eine agonistische Aktivität auf den Kaliumkanal, wenn $\frac{\Delta F}{F}$ größer als $\left(\frac{\Delta F}{F}\right)_K$ ist:

$$\frac{\Delta F}{F} \rangle \left(\frac{\Delta F}{F}\right)_K$$

Um eine echte agonistische Aktivität, von einer normalen Streuung von Messwerten zu unterscheiden, gilt dabei, dass man üblicherweise erst dann auf eine agonistische Aktivität schließen kann, wenn folgende Bedingung erfüllt ist:

$$\frac{\Delta F}{F} \rangle \left[\left(\frac{\Delta F}{F}\right)_K + 3*\sigma\right]$$

$\frac{\Delta F}{F}$ muss außerhalb des so genannten 3σ-Intervalls für die Kontrollzellen liegen, wobei "σ" die Standardabweichung von $\left(\frac{\Delta F}{F}\right)_K$ darstellt.

Im Falle von KCNQ-Kanälen wird bevorzugt nach etwa 60s die Fluoreszenzintensität bestimmt. Je nach Kaliumkanal und Messgerät kann der Zeitpunkt der Bestimmung der Fluoreszenzintensität jedoch unterschiedlich sein. Der geeignete Zeitpunkt kann von einem Fachmann anhand einfacher Routineversuche ermittelt werden. Aus dem Quotienten der vor dem Zusatz der Ionen, für die der Ionenkanal permeabel ist, und nach dem Zusatz dieser Ionen gemessenen Fluoreszenzintensität ergibt sich eine Aussage über die agonistische Aktivität der Zielverbindung auf dem jeweiligen Ionenkanal.

Das erfindungsgemäße Verfahren eignet sich besonders für ein Hochdurchsatzscreening (High Throughput Screening, HTS) für Kaliumkanalöffner. Besonders geeignet ist hierbei die Kultivierung von Zellen in 96 Well Platten, die über entsprechende Fluoreszenzreader ausgelesen werden können. Es ist aber auch eine Anpassung auf 384 oder 1536 Well Platten möglich und damit eine weitere Steigerung des Durchsatzes. Entsprechende Modifikationen sind einem Fachmann bekannt.

Mit dem erfindungsgemäßen Assayformat können hochwirksame Kaliumkanalöffner identifiziert werden. Die hohe Empfindlichkeit des Assayformats erlaubt die Identifizierung von selektiv gezielt auf spezifische Kaliumionenkanäle wirkenden Verbindungen. Das erfindungsgemäße Verfahren eignet sich grundsätzlich zur Untersuchung beliebiger Kaliumkanäle, aber besonders zur Untersuchung der folgenden Kaliumkanäle: spannungsabhängige Kaliumkanäle der $K_V$-Kanalfamilie.

Beispiele für die vorstehenden Kaliumkanäle sind

Spannungsabhängige Kaliumkanäle: $K_V1.1$, $K_V1.2$, $K_V1.3$, $K_V1.4$, $K_V1.5$,. $K_V1.6$, $K_V1.7$, $K_V1.8$, $K_V2.1$, $K_V2.2$, $K_V3.1$, $K_V3.2$, $K_V3.3$, $K_V3.4$, $K_V4.1$, $K_V4.2$, $K_V4.3$, $K_V5.1$, $K_V6.1$, $K_V6.2$, $K_V6.3$, $K_V7.1$, $K_V7.2$, $K_V7.3$, $K_V7.4$, Kv7.5, $K_V8.1$, $K_V9.1$, $K_V9.2$, $K_V9.3$, $K_V10.1$, $K_V10.2$, $K_V11.1$. $K_V11.2$, $K_V11.3$, $K_V12.1$, $K_V12.2$, $K_V12.3$

Bevorzugt eignet sich das erfindungsgemäße Verfahren zur Untersuchung von Kaliumkanälen der $K_V7.x$ - bzw. KCNQ-Familie.

Die Erfindung betrifft ferner die Verwendung des gegebenenfalls modifizierten "ion jump"-Membranpotenzialassays bzw. eines Assays mit den vorstehend angegebenen Schritten zur Identifizierung von Verbindungen mit agonistischer Aktivität auf einen Kaliumionenkanal, insbesondere auf einen spannungsabhängigen Kaliumionenkanal.

Die beigefügten Figuren erläutern die Erfindung näher. Es zeigen

Figur 1: Wirkung von XE991 auf das Membranpotenzial von Zellen, die KCNQ-Kanäle exprimieren, in einem Depolarisationsassayformat. Dargestellt ist der Zeitverlauf der Fluoreszenzintensität von CHO-Zellen, die KCNQ-Kanäle exprimieren, vor und nach der Applikation (Pfeil) eines bekannten KCNQ-Kanalblockers (XE991, 60 $\mu$M).

Figur 2: Wirkung von Retigabine auf das Membranpotenzial von Zellen, die KCNQ-Kanäle exprimieren, in einem Hyperpolarisationsassayformat. Dargestellt ist der Zeitverlauf der Fluoreszenzintensität von CHO-Zellen, die KCNQ-Kanäle exprimieren, vor und nach der Applikation (Pfeil) eines bekannten KCNQ-Kanalöffners (Retigabine, 30 $\mu$M).

Figur 3: Einfluss von XE991 auf die Fluoreszenzantwort nach Kalium-Injektion zum extrazellulären Medium in einem "ion jump" Assayformat zur Suche nach Kaliumkanalblockem. Dargestellt ist der Zeitverlauf der Fluoreszenzintensität von CHO-Zellen, die KCNQ-Kanäle exprimieren, vor und nach Applikation (Pfeil) von 100 mM Kaliumchlorid.

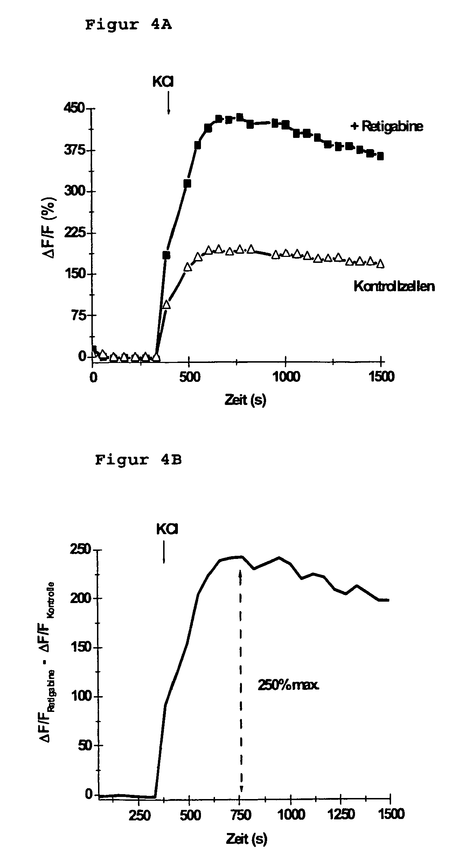

Figur 4: Einfluss von Retigabine auf die Fluoreszenzantwort nach Kalium-Injektion zum extrazellulären Medium in dem "ion jump" Assayformat zur Auffindung von Kaliumkanalöffnern.

**A)** Dargestellt ist der Zeitverlauf der Fluoreszenzintensität von CHO-Zellen, die KCNQ-Kanäle exprimieren, vor und nach Applikation (Pfeil) von 100 mM Kaliumchlorid.
**B)** Differenz der Fluoreszenzverläufe in A.

Figur 5/6/7: Agonistische Aktivität von Retigabine (Figur 5A + B), Substanz A (Figur 6A + B) und Substanz B (Figur 7A + B) in dem "ion jump" Assayformat zur Auffindung von Kaliumkanalöffnern.

**A)** Dargestellt ist der Zeitverlauf der Fluoreszenzintensität von CHO-Zellen, die KCNQ-Kanäle exprimieren, vor und nach Applikation (Pfeil) von 100 mM Kaliumchlorid. Gezeigt sind die Fluoreszenzantworten der Zellen in Anwesenheit verschiedener Konzentrationen von Retigabine (Figur 5A), Substanz A (Figur 6A) und Substanz B (Figur 7A).

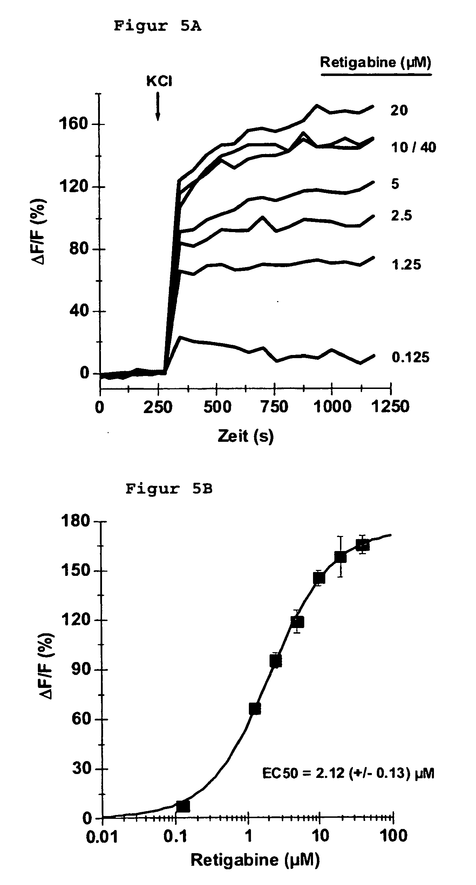

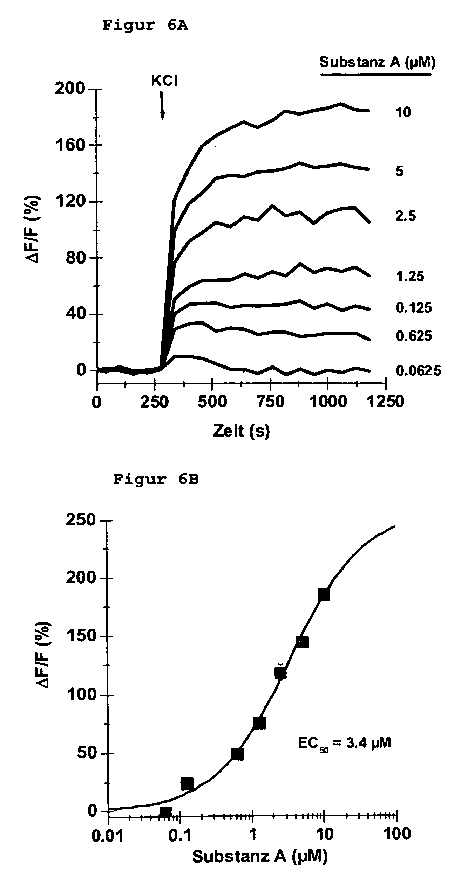

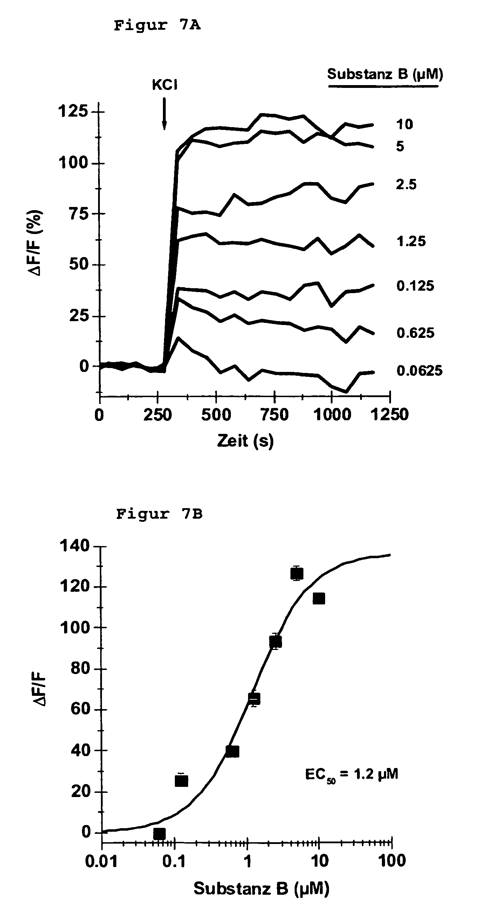

**B)** Dargestellt sind die prozentualen Änderungen der Fluoreszenzintensität ($\Delta F/F$) nach Applikation von 100 mM Kaliumchlorid, in Abhängigkeit der Konzentration von Retigabine (Figur 5B), Substanz A (Figur 6B) und Substanz B (Figur 7B).

Die nachstehenden Beispiele erläutern die Erfindung näher:

**Beispiel 1: Wirkung von XE991 auf das Membranpotenzial von Zellen, die KCNQ-Kanäle exprimieren, in einem Depolarisationsassayformat (Referenzbeispiel)**

Es wurden CHO-Zellen (*chinese hamster ovary cells*), die KCNQ2- und KCNQ3-Kanäle exprimieren, in Nährmedium [Minimum Essential Medium (MEM) $\alpha$ Medium 22571 1x flüssig (Invitrogen,), 10 % Fetal Calf Serum (FCS) (Gibco, heat inactivated), inklusive Selektionsantibiotika] bei 37°C, 5 % $CO_2$ und 95 % Luftfeuchtigkeit in Zellkulturflaschen adhärent kultiviert. Bei 80%iger Konfluenz wurden die Zellen mit 1xDPBS ohne $Ca^{2+}/Mg^{2+}$-Zusatz gewaschen und mit 2 ml Accutase für 15 min bei 37°C inkubiert. Die aus einer T75-Zellkulturflasche abgelösten Zellen wurden in 8 ml Nährmedium resuspendiert. Nach der Bestimmung der Zellzahl in einem CASY Zell-Counter wurden 20.000 Zellen/ well in Corning® CellBIND® 96 Well Platten (Black with clear bottom) in 100 $\mu$l Nährmedium ausgesät und 24h bei 37°C, 5 % $CO_2$ und 95 % Luftfeuchtigkeit inkubiert. Danach erfolgte die Beladung der Zellen mit dem roten FMP-Farbstoff der Firma Molecular Devices. Ein Vial Membrane Potential Assay Kit Red Component A (MPK, Bulk-Kit) wurde in 200 ml extrazellulärer Pufferlösung (ES; 120 mM NaCl, 1 mM KCl, 10 mM HEPES, 2 mM $CaCl_2$, 2 mM $MgCl_2$, 10 mM Glucose; pH 7,4) gelöst. Um die Zellen mit dem spannungssensitiven Fluoreszenzfarbstoff zu beladen, wurde zunächst das Nährmedium verworfen. Die Zellen wurden dann mit 200 $\mu$l ES-Puffer gewaschen, anschließend mit 100 $\mu$l der Farbstofflösung überschichtet und 45 min inkubiert. Nach der Farbstoff-Inkubation wurden entweder 50 $\mu$l ES (Kontrolle) oder in unabhängige wells 50 $\mu$l des KCNQ-Kanalblockers XE991 in sättigender Konzentration (Endkonzentration 60 $\mu$M) zugegeben und 2 h 13 min bei gleichzeitiger Aufnahme der Fluoreszenz im Reader inkubiert. Die Messungen wurden mit einem Fluostar oder Polarstar Fluoreszenzreader der Firma BMG oder der FlexStation der Firma Molecular Devices durchgeführt. Bei einer Wellenlänge von 525 nm wurde angeregt und bei 560 nm detektiert.

In Figur 1 ist der Zeitverlauf der Fluoreszenzintensität von CHO-Zellen, die KCNQ-Kanäle exprimieren, vor und nach der Applikation (Pfeil) eines bekannten KCNQ-Kanalblockers (XE991) dargestellt.

Dargestellt ist die prozentuale Änderung der Fluoreszenzintensität ($\Delta$F/F), bezogen auf die gemittelte Fluoreszenzintensität vor der XE991 Applikation. Der Fluoreszenzverlauf der Kontrollzellen (Applikation von ES-Puffer) wird von dem XE991-induzierten Fluoreszenzsignal subtrahiert.

**Beispiel 2: Wirkung von Retigabine auf das Membranpotenzial von Zellen, die KCNQ-Kanäle exprimieren in einem Hyperpolarisationsassayformat (Vergleichsbeispiel)**

Die Aussaat der Zellen in Assayplatten und die Beladung mit dem Farbstoff erfolgte exakt wie in Beispiel 1 beschrieben. Die Messungen wurden mit einem Fluostar oder Polarstar Fluoreszenzreader der Firma BMG oder der FlexStation der Fa. Molecular Devices durchgeführt. Bei einer Wellenlänge von 525 nm wurde angeregt und bei 560 nm detektiert. Nach der Farbstoff-Inkubation erfolgt die Aufnahme der Basislinie für 5 min. Dann wurden entweder 50 $\mu$l ES-Puffer (Kontrolle) oder 50 $\mu$l des KCNQ-Agonisten Retigabine (30 $\mu$M) zugegeben und 7,5 min die Fluoreszenz im geeigneten Reader aufgenommen.

In Figur 2 ist der Zeitverlauf der Fluoreszenzintensität von CHO-Zellen, die KCNQ-Kanäle exprimieren, vor und nach der Applikation (Pfeil) des bekannten KCNQ-Kanalöffners (Retigabine, 30 $\mu$M) dargestellt. Dargestellt ist die prozentuale Änderung der Fluoreszenzintensität ($\Delta$F/F), bezogen auf die gemittelte Fluoreszenzintensität vor der Retigabine Applikation. Der Fluoreszenzverlauf der Kontrollzellen (Applikation von Puffer) wurde von dem Retigabine-induzierten Fluoreszenzsignal subtrahiert.

**Beispiel 3: Einfluss von XE991 auf die Fluoreszenzantwort nach Kalium-Injektion zum extrazellulären Medium in einem "ion jump" Assayformat zur Suche nach Kaliumkanalblockern**

Die Aussaat der KCNQ-exprimierenden Zellen in Assayplatten, Beladung mit dem Farbstoff und zu verwendende Messgeräte sowie einzustellende Wellenlängen sind gemäß Beispiel 1. Es wurden entweder 50 $\mu$l ES-Puffer (Kontrolle) oder 50 $\mu$l des KCNQ-Blockers XE991 (60 $\mu$M) in unterschiedliche wells einer mit KCNQ-exprimierenden Zellen bestückten Platte zugegeben und 30 min inkubiert. Es erfolgte dann im Fluoreszenzreader die Aufnahme der Basislinie für 3-4 min und die Messung der Fluoreszenzwerte für 10 min nach der Injektion von 15 $\mu$l einer KCl-Lösung (Endkonzentration 91,8 mM) in jede Kavität der Platte.

In Figur 3 ist der Zeitverlauf der Fluoreszenzintensität von CHO-Zellen, die KCNQ-Kanäle exprimieren, vor und nach Applikation (Pfeil) von 91,8 mM Kaliumchlorid dargestellt. Gezeigt sind die Fluoreszenzantworten der Zellen in Abwesenheit ($\Delta$) und Anwesenheit (■) des bekannten KCNQ-Kanalblockers XE991 (60$\mu$M). Dargestellt ist die prozentuale Änderung der Fluoreszenzintensität ($\Delta$F/F), bezogen auf die gemittelte Fluoreszenzintensität vor der Kalium-Applikation.

**Beispiel 4: Einfluss von Retigabine auf die Fluoreszenzantwort nach Kalium-Injektion zum extrazellulären Medium in dem "ion jump" Assayformat zur Auffindung von Kaliumkanalöffnern**

Der Versuch wurde gemäß Beispiel 3 durchgeführt. Es wurden hier allerdings entweder 50 $\mu$l ES-Puffer (Kontrolle) oder 50 $\mu$l des KCNQ-Agonisten Retigabine (30 $\mu$M) in definierte wells einer mit KCNQ-exprimierenden Zellen eingesäten Platte zugegeben und 30 min inkubiert. Es erfolgte dann im Fluoreszenzreader die Aufnahme der Basislinie für 5 min und die Messung der Fluoreszenzwerte für 20 min nach der Injektion von 15 $\mu$l einer KCl-Lösung (Endkonzentration 91,8 mM) in jede Kavität der Platte. Das Ergebnis ist in Figur 4 dargestellt.

**Figur 4A)** Dargestellt ist der Zeitverlauf der Fluoreszenzintensität von CHO-Zellen, die KCNQ-Kanäle exprimieren, vor und nach Applikation (Pfeil) von 91,8 mM Kaliumchlorid. Gezeigt sind die Fluoreszenzantworten der Zellen in Abwesenheit ($\Delta$) und Anwesenheit (■) des bekannten KCNQ-Kanalöffners Retigabine (30 $\mu$M). Dargestellt ist die prozentuale Änderung der Fluoreszenzintensität ($\Delta$F/F), bezogen auf die gemittelte Fluoreszenzintensität vor der Kalium-Applikation.

**Figur 4B**) Differenz der Fluoreszenzverläufe in A. Der Fluoreszenzverlauf in Abwesenheit von Retigabine wurde von dem Fluoreszenzverlauf in Anwesenheit von Retigabine subtrahiert.

**Beispiel 5: Agonistische Aktivität von Retigabine, Substanz A und Substanz B in dem "ion jump" Assayformat zur Auffindung von Kaliumkanalöffnern.**

Für dieses Experiment wurden CHO-Zellen (*chinese hamster ovary cells*), die KCNQ2- und KCNQ3-Kanäle exprimieren, in Nährmedium [Minimum Essential Medium (MEM) $\alpha$ Medium 22571 1x flüssig (Invitrogen,), 10 % Fetal Calf Serum (FCS) (Gibco, heat inactivated), inklusive Selektionsantibiotika] bei 37°C, 5 % $CO_2$ und 95 % Luftfeuchtigkeit in Zellkulturflaschen adhärent kultiviert. Bei 80%iger Konfluenz wurden die Zellen mit 1xDPBS ohne $Ca^{2+}/Mg^{2+}$-Zusatz gewaschen und mit 2 ml Accutase für 15 min bei 37°C inkubiert. Die aus einer T75-Zellkulturflasche abgelösten Zellen wurden in 8 ml Nährmedium resuspendiert. Nach der Bestimmung der Zellzahl in einem CASY Zell-Counter werden 20.000 Zellen/well in Corning® CellBIND® 96 Well Platten (Black with clear bottom) in 100 $\mu$l Nährmedium ausgesät und 24h bei 37°C, 5 % $CO_2$ und 95 % Luftfeuchtigkeit inkubiert. Danach erfolgte die Beladung der Zellen mit dem roten FMP-Farbstoff der Fa. Molecular Devices. Ein Vial Membrane Potential Assay Kit Red Component A (MPK, Bulk-Kit) wurde in 200 ml extrazellulärer Pufferlösung (ES; 120 mM NaCl, 1 mM KCl, 10 mM HEPES, 2 mM $CaCl_2$, 2 mM $MgCl_2$, 10 mM Glucose; pH 7,4) gelöst. Um die Zellen mit dem spannungssensitiven Fluoreszenzfarbstoff zu beladen, wurde zunächst das Nährmedium verworfen. Die Zellen wurden dann mit 200 $\mu$l ES-Puffer gewaschen, anschließend mit 100 $\mu$l der Farbstofflösung überschichtet und 45 min inkubiert. Nach der Farbstoff-Inkubation werden in genau definierte wells der Assayplatte entweder 50 $\mu$l ES-Puffer (Kontrolle) oder 50 $\mu$l der KCNQ-Agonisten Retigabine, Substanz A oder Substanz B zugegeben und für 30 min inkubiert. Die Endkonzentrationen im Ansatz der hier verwandten KCNQ-Öffner sind 0.0625, 0.625, 0.125, 1.25, 2.5, 5 und 10 $\mu$M. Es erfolgte dann im Fluoreszenzreader die Aufnahme der Basislinie für 4-5 min, gefolgt von einer Injektion von 15 $\mu$l einer KCl-Lösung (Endkonzentration 91,8 mM) in jede Kavität der Platte und eine sofortige Weitermessung im Reader für die folgenden 15 min. Die Messungen wurden mit einem Fluostar oder Polarstar Fluoreszenzreader der Firma BMG oder der FlexStation der Firma Molecular Devices durchgeführt. Bei einer Wellenlänge von 525 nm wurde angeregt und bei 560 nm detektiert.
Die Ergebnisse sind in den Figuren 5A + B, 6A + B und 7A + B dargestellt.

A) Dargestellt ist der Zeitverlauf der Fluoreszenzintensität von CHO-Zellen, die KCNQ-Kanäle exprimieren, vor und nach Applikation (Pfeil) von 91,8 mM Kaliumchlorid. Gezeigt sind die Fluoreszenzantworten der Zellen in Anwesenheit verschiedener Konzentrationen von Retigabine (Figur 5A), Substanz A (Figur 6A) und Substanz B (Figur 7A). Die Zellen wurden jeweils mit dem Membrane Potential Kit (Molecular Devices) inkubiert. Dargestellt ist jeweils die prozentuale Änderung der Fluoreszenzintensität ($\Delta$F/F), bezogen auf die gemittelte Fluoreszenzintensität vor der Kalium-Applikation. Der Fluoreszenzverlauf von Kontrollzellen, bei denen kein Wirkstoff appliziert wurde, wurde von den dargestellten Fluoreszenzverläufen subtrahiert.

B) Dargestellt sind die prozentualen Änderungen der Fluoreszenzintensität ($\Delta$F/F) nach Applikation von 91,8 mM Kaliumchlorid, in Abhängigkeit der Konzentration von Retigabine (Figur 5B), Substanz A (Figur 6B) und Substanz B (Figur 7B). $\Delta$F/F wurde jeweils 60s nach Kalium-Applikation ermittelt. Dargestellt sind jeweils Mittelwerte aus drei Messungen. Das $\Delta$F/F für die Kontrollzellen (in Abwesenheit von Wirkstoff) wurde subtrahiert. An die Datenpunkte wurde mit Hilfe der Hill-Gleichung eine Dosis-Wirkungs-Kurve angepasst und so die angegebenen EC50-Werte für die agonistische Aktivität ermittelt

**Beispiel 6: Untersuchung von KCNQ-Agonisten**

Die folgende Versuchsdurchführung beschreibt ein bevorzugtes Protokoll, mit dem spannungssensitive Kaliumkanäle (dargestellt am Beispiel der KCNQ-Kanäle) im Hinblick auf Kanalöffner untersucht werden können. Mit diesem Protokoll können alle Arten von Kaliumkanalöffnern bzw. Kaliumkanalagonisten untersucht werden. Dabei können die vorstehend genannten Fluoreszenzfarbstoffe verwendet werden.
Um die CHO-Zellen (*chinese hamster ovary cells*), die KCNQ-Kanäle exprimieren, zu kultivieren werden sie in Minimum Essential Medium (MEM) $\alpha$ Medium 22571 1x flüssig (Invitrogen,) mit 10 % Fetal Calf Serum (FCS) (Gibco, heat inactivatetd) und Selektionsantibiotika bei 37°C, 5 % $CO_2$ und 95 % Luftfeuchtigkeit adherent kultiviert. Dabei werden TC Flask 80 $cm^2$ (Nunc) als Zellkulturflaschen verwendet.
Die Zellen werden passagiert in dem man in einem ersten Schritt das Kulturmedium abgießt und die Zellen anschließend mit einem Puffer einer Zusammensetzung von 0,9 mM $CaCl_2$, 2,7 mM KCl, 1,5 mM $KH_2PO_4$, 0,5 mM $MgCl_2$, 140 mM NaCl und 8 mM $Na_2HPO_4$ spült. Um die Zellen von der Kulturflasche abzulösen werden 2 ml Accutase (PAA Laboratories) zugegeben. Man inkubiert 15 Minuten bei 37°C dadurch beginnen sich die Zellen abzukugeln. Die Zellen werden durch Anschlagen mit der flachen Hand an den Kulturgefäßrand von Boden der Zellkulturflaschen gelöst.

Die Bestimmung der vorliegenden Zellzahl wird mit einem CA-SY Model TCC Zellzählgerät (Schärfe System) durchgeführt. Die Aussaat der Zellen in neue Kulturgefäße erfolgt in 20 ml Medium. Dabei werden folgende Zellzahlen eingesät (A = Tag der Zellaussaat, E= Tag der Zellernte): A: Montag - E: Donnerstag → $4 \cdot 10^5$ Zellen; A: Montag - E: Freitag → $3 \cdot 10^5$ Zellen; A: Donnerstag - E: Montag → $3 \cdot 10^5$ Zellen; A: Freitag - E: Dienstag → $3 \cdot 10^5$ Zellen; A: Freitag - E: Mittwoch → $2 \cdot 10^5$ Zellen.

Zur Aussaat der Zellen in 96 Well Messplatten werden die Zellen wie oben beschrieben gewaschen und von dem Kulturgefäß abgelöst. Nach dem Bestimmen der vorliegenden Zellzahl werden 20.000 Zellen/well in Corning® CellBIND® 96 Well Platten (Black with clear bottom) in 100 $\mu$l des beschriebenen Nährmediums ausgesät, 1 h bei Raumtemperatur ohne Begasung oder Regelung der Luftfeuchtigkeit und 24h bei 37°C, 5 % $CO_2$ und 95 % Luftfeuchtigkeit inkubiert.

Um die Zellen mit dem verwendeten spannungssensitiven Fluoreszenzfarbstoff beladen zu können wird ein *Vial Membrane Potential Assay Kit* Red Component A (MPK, Bulk-Kit) in 200 ml extrazellulärer Lösung (ES; 120 mM NaCl, 1 mM KCl, 10 mM HEPES, 2 mM $CaCl_2$, 2 mM $MgCl_2$, 10 mM Glucose; pH 7,4) gelöst. Das die Zellen überschichtende Nährmedium wird verworfen. Die Zellen werden mit 200 $\mu$l ES-Puffer gewaschen, anschließend mit 100 $\mu$l der Farbstofflösung überschichtet und im Dunkeln 45 min bei Raumtemperatur ohne Begasung oder Regelung der Luftfeuchtigkeit inkubiert.

Die Fluoreszenzmessungen werden mit einem Fluostar Fluoreszenzreader (BMG) durchgeführt. Die Fluoreszenz wird bei 525 nm angeregt und bei 560 nm detektiert. Nach der Farbstoff-Inkubation wird die zu testende Substanz in der gewünschten Konzentration in 50 $\mu$l Volumen oder zur Kontrolle 50 $\mu$l ES zugegeben und 30 min inkubiert. Anschließend nimmt man die Fluoreszenzintensität des Farbstoffs in den Wells 5 min auf. Daraufhin erfolgt eine zweite Injektion von 15 $\mu$l einer 100 mM KCl-Lösung (Endkonzentration 91,8 mM). Die Veränderung der Fluoreszenz wird für 30 min verfolgt um alle relevanten Messwerte zu erhalten.

## Patentansprüche

1. Verfahren zur Identifizierung der agonistischen Aktivität einer Zielverbindung auf einen spannungsabhängigen Kaliumkanal, **dadurch gekennzeichnet, dass** man

   a) eine Population von Zellen, die besagten Kaliumkanal exprimieren, bereitstellt,
   b) die Zellen gemäß a) mit einem spannungssensitiven Fluoreszenzfarbstoff inkubiert,
   c) die Zielverbindung dem Reaktionsansatz von b) zusetzt,
   d) einen Wert $F_1$ der Fluoreszenzintensität der Zellen bestimmt,
   d) Kaliumionen in einer physiologisch verträglichen Konzentration zusetzt,
   f) einen Wert $F_2$ der Fluoreszenzintensität der Zellen bestimmt, und
   g) die Fluoreszenzintensität $F_2$ mit der Fluoreszenzintensität $F_1$ vergleicht und daraus die agonistische Aktivität der Zielverbindung auf den Kaliumkanal ermittelt.

2. Verfahren nach Anspruch 1, **dadurch gekennzeichnet, dass** die Population von Zellen, die einen Kaliumkanal exprimiert zusätzlich einen Protein-basierten fluoreszenzoptischen Spannungssensor exprimiert, Schritt b) entfällt und die Zielverbindung dem Reaktionsansatz von a) zugesetzt wird.

3. Verfahren nach Anspruch 1 oder 2, **dadurch gekennzeichnet, dass** der spannungsabhängige Kaliumkanal aus der Familie der Kv-Kanäle ausgewählt ist.

4. Verfahren nach Anspruch 3, **dadurch gekennzeichnet, dass** der spannungsabhängige Kaliumkanal aus der Familie der $K_v$-Kanäle ein Kanal der $K_v$7.x - bzw. KCNQ-Familie ist.

5. Verfahren nach einem der Ansprüche 1 bis 4, **dadurch gekennzeichnet, dass** die Population von Zellen, die den Kaliumkanal exprimieren, aus transient transfizierten Zellen, stabilen Zelllinien, primären Zellkulturen oder Gewebszellen oder Zellen, die endogen einen Kaliumkanal exprimieren, ausgewählt ist.

6. Verfahren nach einem der Ansprüche 1 bis 5, **dadurch gekennzeichnet, dass** der Fluoreszenzfarbstoff ein spannungssensitiver Fluoreszenzfarbstoff ist.

7. Verfahren nach Anspruch 6, **dadurch gekennzeichnet, dass** der spannungssensitive Fluoreszenzfarbstoff ausgewählt ist aus langsamen oder schnellen Farbstoffen, FRET-basierten Farbstoffkombinationen, oder Protein-basierten fluoreszenzoptischen Spannungssensoren.

8. Verfahren nach Anspruch 7, **dadurch gekennzeichnet, dass** der spannungssensitive Fluoreszenzfarbstoff ein schneller spannungssensitiver Farbstoff, ausgewählt aus Styrylfarbstoffen, Oxonolfarbstoffen oder Hemicyaninfarbstoffen, ist.

9. Verfahren nach Anspruch 8, **dadurch gekennzeichnet, dass** der spannungssensitive Farbstoff ein langsamer spannungssensitiver Fluoreszenzfarbstoff, ausgewählt aus Dis $BAC_2(3)$-, Di $BAC_4(3)$-, Di $OC_2(3)$-, Dis $C_3(3)$-, Dis $C_3(5)$-Farbstoffen, FRET-basierten Farbstoffsystemen enthaltend Dis $BAC_2(3)$ oder Dis $BAC_4(3)$ und das membrangebundene Coumarin-Phospholipid $CC_2$-DM PE oder enthaltend Dis $BAC,(3)$ / Dis $BAC_1(5)$, Carbocyaninfarbstoffen, Rhodaminfarbstoffen, Oxonol- und Bisoxonolfarbstoffen oder Merocyaninfarbstoffen ist.

10. Verfahren nach einem der Ansprüche 1 bis 9, **dadurch gekennzeichnet, dass** das Verfahren einer Verwendung im HochdurchsatzScreening angepasst ist.

**Claims**

1. Method for identifying the agonistic activity of a target compound on a voltage-dependent potassium channel **characterized in that**

   a) a population of cells expressing said potassium channel is provided,
   b) the cells according to a) are incubated with a voltage-sensitive fluorescent dye,
   c) the target compound is added to the reaction batch of b),
   d) a value $F_1$ of the fluorescence intensity of the cells is determined,
   e) potassium ions in a physiologically acceptable concentration are added,
   f) a value $F_2$ of the fluorescence intensity of the cells is determined, and
   g) the fluorescence intensity $F_2$ is compared with the fluorescence intensity $F_1$ and the agonistic activity of the target compound on the potassium channel is determined therefrom.

2. Method according to claim 1 **characterized in that** the population of cells expressing a potassium channel additionally expresses a protein-based fluorescence-optical voltage sensor, step b) is omitted and the target compound is added to the reaction batch of a).

3. Method according to claim 1 or 2 **characterized in that** the voltage-dependent potassium channel is selected from the family of the $K_v$ channels.

4. Method according to claim 3 **characterized in that** the voltage-dependent potassium channel from the family of the $K_v$ channels is a channel of the $K_v$7.x family and KCNQ family, respectively.

5. Method according to any one of claims 1 to 4 **characterized in that** the population of cells expressing the potassium channel is selected from transiently transfected cells, stable cell lines, primary cell cultures or tissue cells or cells endogenously expressing a potassium channel.

6. Method according to any one of claims 1 to 5 **characterized in that** the fluorescent dye is a voltage-sensitive fluorescent dye.

7. Method according to claim 6 **characterized in that** the voltage-sensitive fluorescent dye is selected from slow or fast dyes, FRET-based combinations of dyes, or protein-based fluorescence-optical voltage sensors.

8. Method according to claim 7 **characterized in that** the voltage-sensitive fluorescent dye is a fast voltage-sensitive dye selected from styryle dyes, oxonol dyes or hemicyanine dyes.

9. Method according to claim 8 **characterized in that** the voltage-sensitive dye is a slow voltage-sensitive fluorescent dye selected from Dis $BAC_2(3)$, Di $BAC_4(3)$, Di $OC_2(3)$, Dis $C_3(3)$, Dis $C_3(5)$ dyes, FRET-based dye systems containing Dis $BAC_2(3)$ or Dis $BAC_4(3)$ and the membrane-bound coumarin phospholipid $CC_2$-DM PE or containing Dis $BAC_1(3)$/Dis $BAC_1(5)$, carbocyanine dyes, rhodamine dyes, oxonol and bisoxonol dyes, or merocyanine dyes.

10. Method according to any one of claims 1 to 9 **characterized in that** the method is adapted to a use in the high-throughput screening.

**Revendications**

1. Procédé d'identification de l'activité agonistique d'un composé cible sur un canal potassique tensiodépendant, **caractérisé en ce que** l'on

   a) met à disposition une population de cellules qui expriment ledit canal potassique,
   b) incube les cellules selon a) avec un colorant par fluorescence tensiosensible,
   c) ajoute le composé cible à la préparation réactionnelle du b),
   d) détermine une valeur $F_1$ de l'intensité de fluorescence des cellules,
   e) ajoute les ions potassium en une concentration physiologiquement acceptable,
   f) détermine une valeur $F_2$ de l'intensité de fluorescence des cellules, et
   g) compare l'intensité de fluorescence $F_2$ à l'intensité de fluorescence $F_1$ et l'on en déduit l'activité agonistique du composé cible sur le canal potassique.

2. Procédé selon la revendication 1, **caractérisé en ce que** la population de cellules exprimant un canal potassique exprime en plus un détecteur de tension par optique de fluorescence à base de protéine, l'étape b) est ignorée, et le composé cible est ajouté à la préparation réactionnelle du a).

3. Procédé selon la revendication 1 ou 2, **caractérisé en ce que** le canal potassique tensiodépendant est choisi dans la famille des canaux $K_v$.

4. Procédé selon la revendication 3, **caractérisé en ce que** le canal potassique tensiodépendant de la famille des canaux $K_v$ est un canal de la famille $K_v$7x ou KCNQ.

5. Procédé selon l'une des revendications 1 à 4, **caractérisé en ce que** la population de cellules exprimant le canal potassique est choisie parmi des cellules transitoirement transfectées, des lignées cellulaires stables, des cultures de cellules primaires, des cellules de tissu ou des cellules qui expriment de manière endogène un canal potassique.

6. Procédé selon l'une des revendications 1 à 5, **caractérisé en ce que** le colorant de fluorescence est un colorant de fluorescence tensiosensible.

7. Procédé selon la revendication 6, **caractérisé en ce que** le colorant de fluorescence tensiosensible est choisi parmi des colorants lents ou rapides, des combinaisons de colorants à base FRET, ou les détecteurs de tension par optique de fluorescence à base de protéines.

8. Procédé selon la revendication 7, **caractérisé en ce que** le colorant de fluorescence tensiosensible est un colorant tensiosensible rapide, choisi parmi les colorants styryle, les colorants oxonol ou les colorants hémicyanine.

9. Procédé selon la revendication 8, **caractérisé en ce que** le colorant tensiosensible est un colorant de fluorescence tensiosensible lent, choisi parmi les colorants Dis $BAC_2$(3), Di $BAC_4$(3), Di $OC_2$(3), Dis $C_3$(3), Dis $C_3$(5), les systèmes de colorants à base FRET contenant Dis Bac(3) ou Dis $BAC_4$(3) et le coumarine-phospholipide lié à la membrane $CC_2$-DMPE ou contenant Dis $BAC_1$(3)/Dis $BAC_1$(5), les colorants carbocyanine, les colorants rhodamine, les colorants oxonol et bisoxonol ou les colorants mérocyanine.

10. Procédé selon l'une des revendications 1 à 9, **caractérisé en ce que** le procédé est adapté à une utilisation dans le criblage haut débit.

Figur 1

Figur 2

Figur 3

Figur 4A

Figur 4B

Figur 5A

Figur 5B

Figur 6A

Figur 6B

## Figur 7A

## Figur 7B

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6329367 B
- WO 0232419 A
- EP 1760158 A


**In der Beschreibung aufgeführte Nicht-Patentliteratur**

- **FORD et al.** *Progress in Drug Research,* 2002, vol. 58, 133 ff
- **WOLFF et al.** Comparative Study of Membrane Potential-Sensitive Fluorescent Probes and Their Use in Ion Channel Screening Assays. *Journal of Biomolecular Screening,* 2003, vol. 8 (5
- **SCOTT et al.** *Analytical Biochemistry,* 2003, vol. 319, 251-257
- **WHITEAKER et al.** *Journal of Biomolecular Screening,* 2001, vol. 6 (5
- **FALCONER et al.** *Journal of Biomolecular Screening,* 2002, vol. 7 (5
- **WOLFF et al.** *Journal of Biomolecular Screening,* 2003, vol. 8 (5
- **GOPALAKRISHNAN et al.** *The Journal of Pharmacology and Experimental Therapeutics,* 2002, vol. 303 (1), 379-386
- *British Journal of Pharmacology,* 2004, vol. 143, 81-90